# EUROPEAN PATENT SPECIFICATION

(11) **EP 1 192 170 B1**
(45) Date of publication and mention of the grant of the patent: **14.05.2003**
(21) Application number: 00946831.5
(22) Date of filing: 21.06.2000
(51) Int. Cl.: C07H 19/16, A61K 31/7076, A61P 9/00

(54) **PROPARGYL PHENYL ETHER A2A RECEPTOR AGONISTS**
PROPARGYLPHENYL-ÄTHER A2A REZEPTORAGONISTEN
AGONISTES DU RECEPTEUR A2A DE PROPARGYL PHENYL ETHER

(30) Priority: 22.06.1999 US 338183
(43) Date of publication of application: 03.04.2002
(73) Proprietor: CV THERAPEUTICS, INC., Palo Alto, CA 94304 (US)
(72) Inventor: ZABLOCKI, Jeff, A., Mountain View, CA 94040 (US); ELZEIN, Elfatih, O., Fremont, CA 94555 (US); PALLE, Venkata, P., Mountain View, CA 94041 (US); NUDELMAN, Gregory, Pacifica, CA 94044 (US)
(74) Representative: Vossius, Volker, Dr.
(86) International application number: PCT/US00/17001
(87) International publication number: WO 00/078777

(56) References cited:
- WO-A-00/23447
- WO-A-96/28163
- WO-A-98/08855
- CHEMICAL ABSTRACTS, vol. 131, no. 24, 13 December 1999 (1999-12-13) Columbus, Ohio, US; abstract no. 317333, KLOTZ K-N. ET AL: "2-Substituted N-ethylcarboxamidoadenosine derivatives as high affinity agonists at human A3 adenosine receptors" XP002150133 & NAUNYN-SCHMIEDEBERG'S ARCH. PHARMACOL., vol. 360, no. 2, 1999, pages 103-108,
- KNUTSEN L J S ET AL: "THE SYNTHESIS AND BIOCHEMICAL EVALUATION OF NEW A1 SELECTIVE ADENOSINE RECEPTOR AGONISTS CONTAINING 6-HYDRAZINOPURINE MOIETIES" BIOORGANIC & MEDICINAL CHEMISTRY LETTERS,GB,OXFORD, vol. 3, no. 12, 1993, pages 2661-2666, XP000870169 ISSN: 0960-894X

## Description

### Background Of The Invention

### Field of Invention

This invention includes 2-adenosine propargyl phenyl ether compounds that are useful as A_{2A} receptor agonists. The compounds of this invention are vasodialating agents that are useful as heart imaging agents that aid in the identification of mammals, and especially humans who are suffering from disorders such poor coronary perfusion which is indicative of coronary artery disease (CAD). The compounds of this invention can also be used as therapeutics for coronary artery disease.

### Description of the Art

Pharmacological stress is frequently induced with adenosine or dipyridamole in patients with suspected CAD before imaging with T1 scintigraphy or echocardiography. Both drugs effect dilation of the coronary resistance vessels by activation of cell surface A₂ receptors. Although pharmacological stress was originally introduced as a mean of provoking coronary dilation in patients unable to exercise, several studies have shown that the prognostic value of ²⁰¹T1 or echocardiographic imaging in patients subjected to pharmacological stress with adenosine or dipyridamole was equivalent to patients subjected to traditional exercise stress tests. However, there is a high incidence of drug-related adverse side effects during pharmacological stress imaging with these drugs such as headache and nausea, that could be improved with new therapeutic agents.

Adenosine A_{2B} and A₃ receptors are involved in a mast cell degranulation and, therefore, asthmatics are not given the non-specific adenosine agonists to induce a pharmacological stress test. Additionally, adenosine stimulation of the A₁ receptor in the atrium and A-V mode will diminish the S-H interval which can induce AV block. (N.C. Gupto et al.; *J*. *Am Coll. Cardiol;* (1992) 19: 248-257). Also, stimulation of the adenosine A1 receptor by adenosine may be responsible for the nausea since the A₁ receptor is found in the intestinal tract. (J. Nicholls et al.; *Eur. J. Pharm.*(1997) 338(2) 143-150).

Animal data suggests that specific adenosine A_{2A} subtype receptors on coronary resistance vessels mediate the coronary dilatory responses to adenosine, whereas subtype A_{2B} receptor stimulation relaxes peripheral vessels (note: the latter lowers systemic blood pressure). As a result there is a need for pharmaceutical compositions that are A_{2A} receptor agonists that have no pharmacological effect as a result of stimulating the A₁ receptor *in vivo.* Furthermore, there is a need for A_{2A} receptor agonists that have a short half-life, and that are well tolerated by patients undergoing pharmacological coronary stress evaluations.

### SUMMARY OF THE INVENTION

In one aspect, this invention relates to 2-adenosine propargyl phenyl ether compounds that are useful A_{2A} receptor agonists.

In another aspect, this invention relates to pharmaceutical compositions including 2-adenosine propargyl phenyl ether compounds that are well tolerated with few side effects.

Still another aspect of this invention are 2-adenosine propargyl phenyl ether compounds that can be easily used in conjunction with radioactive imaging agents to facilitate coronary imaging.

In one embodiment, this invention relates to 2-adenosine propargyl phenyl ether compounds having the following formula:

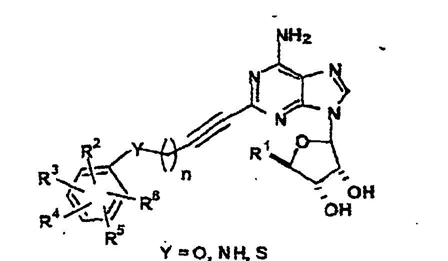

The compounds of this invention stimulate coronary vasodilatation in mammals, and especially in humans, for stressing the heart induced steal situation for purposes of imaging the heart.

In still another embodiment, this invention includes pharmaceutical compositions of matter comprising one or more compounds of this invention and one or more pharmaceutical excipients.

### DESCRIPTION OF THE CURRENT EMBODIMENT

The 2-adenosine propargyl phenyl ether compounds have the following general formula: 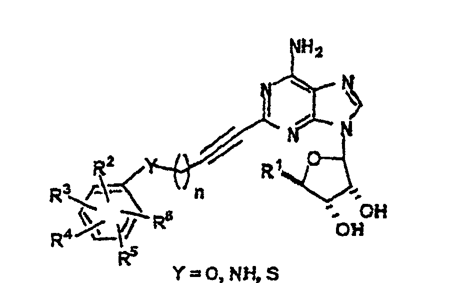 wherein
n is 1 or 2;
Y is O, NH and S;
R¹ is CH₂OH, or C(=O)NR⁷R⁸;
R², R³, R⁴, R⁵, and R⁶ are each individually selected from the group consisting of hydrogen, halo, NO₂, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂ NR²⁰COR²², NR²⁰CO₂R²², NR²⁰CON(R²⁰)₂, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰, OCON(R²⁰)₂, C₁₋₁₅ alkyl, C₂₋₁₅ alkenyl, C₂₋₁₅ alkynyl, heterocyclyl, aryl, and heteroaryl, which alkyl, alkenyl, alkynyl, C₁₋₁₅ alkoxy, aryl, heterocyclyl, and heteroaryl are optionally substituted with from 1 to 3 substituents independently selected from the group consisting of halo, NO₂, heterocyclyl, aryl, heteroaryl, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂ NR²⁰COR²², NR²⁰CO₂R²², NR²⁰CON(R²⁰)₂, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰, and OCON(R²⁰)₂ and wherein each optional heteroaryl, aryl, and heterocyclyl substituent is further optionally substituted with halo, NO₂, alkyl, CF₃, amino, mono- or di- alkylamino, alkyl or aryl or heteroaryl amide, NCOR²², NR²⁰SO₂R²², COR²⁰, CO₂R²⁰, CON(R²⁰)₂, NR²⁰CON(R²⁰)₂, OC(O)R²⁰, OC(O)N(R²⁰)₂, SR²⁰, S(O)R²², SO₂R²², SO₂N(R²⁰)₂, CN, or OR²⁰;
R⁷ and R⁸ are each independently selected from H, and C₁₋₁₅ alkyl optionally substituted with from 1 to 2 substituents independently selected from the group consisting of halo, NO₂, heterocyclyl, aryl, heteroaryl, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂ NR²⁰COR²², NR²⁰CO₂R²², NR²⁰CON(R²⁰)₂, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰, and OCON(R²⁰)₂ and each optional heteroaryl, aryl, and heterocyclyl substituent is further optionally substituted with halo, NO₂, alkyl, CF₃, amino, monoalkylamino or dialkylamino, alkylamide, arylamide or heteroarylamide, NCOR²², NR²⁰SO₂R²², COR²⁰, CO₂R²⁰, CON(R²⁰)₂, NR²⁰CON(R²⁰)₂, OC(O)R²⁰, OC(O)N(R²⁰)₂, SR²⁰, S(O)R²², SO₂R²², SO₂N(R²⁰)₂, CN, and OR²⁰;
R²⁰ is selected from the group consisting of H, C₁₋₁₅ alkyl, C₂₋₁₅ alkenyl, C₂₋₁₅ alkynyl, heterocyclyl, aryl, and heteroaryl, which alkyl, alkenyl, alkynyl, heterocyclyl, aryl, and heteroaryl are each optionally substituted with from 1 to 3 substituents independently selected from halo, alkyl, mono- or dialkylamino, alkyl or aryl or heteroaryl amide, CN, O-C₁₋₆ alkyl, CF₃, aryl, and heteroaryl; and
R²² is selected from the group consisting of C₁₋₁₅ alkyl, C₂₋₁₅ alkenyl, C₂₋₁₅ alkynyl, heterocyclyl, aryl, and heteroaryl which alkyl, alkenyl, alkynyl, heterocyclyl, aryl, and heteroaryl are each optionally substituted with from 1 to 3 substituents independently selected from halo, alkyl, mono- or dialkylamino, alkyl or aryl or heteroaryl amide, CN, -O-C₁₋₆ alkyl, CF₃, and heteroaryl.

In alternative embodiment, this invention is a compound having the formula identified above wherein R², R³, R⁴, R⁵, and R⁶ are each individually selected from the group consisting of hydrogen, halo, NO₂; CF₃, CN, OR²⁰, SR²⁰, N(R²⁰)₂, S(O)R²², SO₂R²², SO₂N(R²⁰)₂, NR²⁰CO₂R²², NR²⁰CON(R²⁰)₂, COR²⁰, CO₂R²⁰, CON(R²⁰)₂, NR²⁰SO₂R²², OC(O)R²⁰, OCON(R²⁰)₂, C₁₋₁₅ alkyl, C₂₋₁₅ alkenyl, C₂₋₁₅ alkynyl, heterocyclyl, aryl, and heteroaryl, wherein the alkyl, alkenyl, alkynyl, aryl, heterocyclyl, and heteroaryl are each optionally substituted with from 1 to 3 substituents independently selected from the group consisting of halo, NO₂, heterocyclyl, aryl, heteroaryl, CF₃, CN, OR²⁰, SR²⁰, N(R²⁰)₂, S(O)R²², SO₂R²², SO₂N(R²⁰)₂, NR²⁰CO₂R²², NR²⁰CON(R²⁰)₂, COR²⁰, CO₂R²⁰, CON(R²⁰)₂, NR²⁰SO₂R²², OC(O)R²⁰, and OCON(R²⁰)₂ with each optional heteroaryl, aryl, and heterocyclyl substituent further optionally substituted with halo, alkyl, CF₃, mono- or di-alkylamino, SR²⁰, CN, and OR²⁰; R⁷ and R⁸ are each individually selected from H, and C₁₋₁₅ alkyl optionally substituted with from 1 to 2 substituents independently selected from the group consisting of aryl, heteroaryl, CF₃, and OR²⁰, wherein each optional heteroaryl or aryl substituent is optionally substituted with halo, alkyl, and CF₃; and R²⁰ is selected from the group consisting of H, C₁₋₆ alkyl, C₂₋₆ alkenyl, C₂₋₆ alkynyl, and aryl wherein each alkyl, alkenyl, alkynyl, and aryl are further optionally substituted with from 1 to 3 substituents independently selected from halo, alkyl, CN, O-C₁₋₆ alkyl, and CF₃.

In another alternative embodiment, the compound of this invention has the formula identified above wherein R², R³, R⁴, R⁵, and R⁶ are individually selected from the group consisting of hydrogen, halo, NO₂, CF₃, CN, OR²⁰, SR²⁰, N(R²⁰)₂, C₁₋₁₅ alkyl, C₂₋₁₅ alkenyl, heterocyclyl, aryl, and heteroaryl which alkyl, alkenyl, aryl, heterocyclyl, and heteroaryl are optionally substituted with from 1 to 3 substituents independently selected from the group consisting of halo, NO₂, aryl, CF₃, CN, OR²⁰, SR²⁰, N(R²⁰)₂, with each optional aryl, substituend further optionally substituted with halo, alkyl, CF₃, SR²⁰, CN, and OR²⁰; R⁷ and R⁸ are each independently selected from H and C₁₋₁₅ alkyl substituted with from 1 to 2 CF₃ groups and R²⁰ is selected from the group consisting of H, and C₁₋₄ alkyl, wherein the C₁₋₄ alkyl is optionally substituted with from 1 to 3 substituents independently selected from halo, alkyl, CN, O-C₁₋₃ alkyl, and CF₃.

In yet another alternative embodiment, the compound of this invention has the formula identified above wherein R², R³, R⁴, R⁵, and R⁶ are each individually selected from the group consisting of hydrogen, halo, CF₃, CN, OR²⁰, SR²⁰, N(R²⁰)₂, C₁₋₁₅ alkyl, heterocyclyl, and aryl, wherein the alkyl, aryl, and heterocyclyl are optionally substituted with 1 to 2 substituents independently selected from the group consisting of halo, aryl, CF₃, CN, OR²⁰, SR²⁰, N(R²⁰)₂, and wherein each optional aryl substituent is further optionally substituted with halo, alkyl, CF₃, CN, and OR²⁰; R⁷ and R⁸ each individually selected from H, and C₁₋₁₅ alkyl; and R²⁰ is selected from the group consisting of H, and C₁₋₃ alkyl, wherein the C₁₋₃ alkyl is optionally substituted with from 1 to 3 substituents independently selected from halo, alkyl, CN, and CF₃.

In still another alternative embodiment, the compound of this invention has the formula identified above wherein R², R³, R⁴, R⁵, and R⁶ are each individually selected from the group consisting of hydrogen, halo, CF₃, CN, OR²⁰, SR²⁰, and N(R²⁰)₂, C₁₋₁₅ alkyl, heterocyclyl, and aryl, wherein the alkyl, aryl, and heterocyclyl are optionally substituted with from 1 to 2 substituents independently selected from the group consisting of halo, aryl, CF₃, CN, OR²⁰, SR²⁰, and N(R²⁰)₂, and wherein each optional aryl substituent is further optionally substituted with halo, alkyl, CN, and CF₃; R⁷ and R⁸ are each individually selected from H, and C₁₋₈ alkyl; and R²⁰ is selected from the group consisting of H, and C₁₋₃ alkyl. In this embodiment, it is preferred that at least three substituents selected from the group consisting of R², R³, R⁴, R⁵, and R⁶ are hydrogen, wherein Y is O and wherein n is 1. In this embodiment, it is more preferred that R¹ is CH₂OH; R², R³, R⁴, R⁵, and R⁶ are each individually selected from the group consisting of hydrogen, halo, CF₃, CN, OR²⁰, C₁₋₈ alkyl, heterocyclyl, and aryl, wherein the alkyl, aryl, and heterocyclyl are optionally substituted with one substituent selected from the group consisting of halo, aryl, CF₃, CN, and OR²⁰, and wherein each optional aryl substituent is further optionally substituted with halo, alkyl, CN, and CF₃; and R²⁰ is selected from the group consisting of H, and C₁₋₃ alkyl. Alternatively, in this embodiment, it is preferred that R¹ is CH₂OH; R², R³, R⁴, R⁵, and R⁶ are each individually selected from the group consisting of hydrogen, halo, CF₃, CN, OR²⁰, C₁₋₆ alkyl, heterocyclyl, and aryl, wherein the alkyl, and aryl are optionally substituted with one substituent selected from the group consisting of halo, aryl, CF₃, CN, and OR²⁰; and R²⁰ is selected from the group consisting of H, and C₁₋₃ alkyl. In yet another alternative of this embodiment, it is preferred that R¹ is CH₂OH; R², R³, R⁴, R⁵, and R⁶ are each individually selected from the group consisting of hydrogen, halo, CF₃, CN, OR²⁰, C₁₋₄ alkyl optionally substituted with aryl and heterocyclyl wherein the heterocyclyl is a fused five to seven membered ring containing 1 to 3 heteroatoms selected from O, N, S, and combinations thereof; and R²⁰ is selected from the group consisting of H, and C₁₋₃ alkyl. In still another alternative to this embodiment, it is preferred that R¹ is CH₂OH; R², R³, R⁴, R⁵, and R⁶ are each individually selected from the group consisting of hydrogen, halo, CF₃, CN, OR²⁰, C₁₋₄ alkyl and aryl optionally substituted with aryl and heterocyclyl wherein the heterocyclyl is a fused five membered ring containing two oxygen atoms; and R²⁶ is selected from the group consisting of H, and C₁₋₃ alkyl. In still another alternative to this embodiment, it is preferred that R¹ is CH₂OH, and R², R³, R⁴, R⁵, and R⁶ are each individually selected from the group consisting of hydrogen, CN, and C₁₋₄ alkyl optionally substituted with aryl and a fused five membered ring containing two oxygen atoms with points of attachments at the 2 and 3 position. In still another alternative to this embodiment, it is preferred that R¹ is C(O)NHEt; R², R³, R⁴, R⁵, and R⁶ are each individually selected from the group consisting of hydrogen, halo, CF₃, CN, OR²⁰, C₁₋₃ alkyl, heterocyclyl, and aryl wherein the alkyl, aryl, and heterocyclyl are optionally substituted with a substituent independently selected from the group consisting of halo, aryl, CF₃, CN, and OR²⁰, wherein each optional aryl substituent is further optionally substituted with halo, alkyl, CN, and CF₃; and R²⁰ is selected from the group consisting of H, and C₁₋₃ alkyl. In a further alternative to this embodiment, it is preferred that R¹ is C(O)NHEt; R², R³, R⁴, R⁵, and R⁶ are each individually selected from the group consisting of hydrogen, halo, CF₃, CN, OR²⁰, C₁₋₆ alkyl, heterocyclyl, and aryl, wherein the alkyl and aryl are optionally substituted with one substituent selected from the group consisting of halo, aryl, CF₃, CN, and OR²⁰; and R²⁰ is selected from the group consisting of H, and C₁₋₃ alkyl. In still a further alternative to this embodiment, it is preferred that R¹ is C(O)NHEt; R², R³, R⁴, R⁵, and R⁶ are each individually selected from the group consisting of hydrogen, halo, CF₃, CN, OR²⁰, aryl, C₁₋₄ alkyl, optionally substituted with one substituent selected from aryl, and a fused five to seven membered ring containing 1-3 heteroatoms selected from O, N, S; and R²⁰ is selected from the group consisting of H, and C₁₋₃ alkyl. is yet a further alternative of this embodiment, it is preferred that R¹ is C(O)NHEt; R², R³, R⁴, R⁵, and R6 are each individually selected from the group consisting of hydrogen, halo, CF₃, CN, OR²⁰, aryl, and C₁₋₄ alkyl that is optionally substituted with one substituent selected from the group consisting of aryl and a fused five membered ring containing two oxygen atoms; and R²⁰ is selected from the group consisting of H, and C₁₋₃ alkyl. In a final alternative of this embodiment it is preferred that R¹ is C(O)NHEt; and R², R³, R⁴, R⁵, and R⁶ are each individually selected from the group consisting of hydrogen, CN, aryl, and C₁₋₄ alkyl that is optionally substituted with one substituent selected from aryl, and a fused five membered ring containing two oxygen atoms with points of attachment at the 2 and 3 position.

Preferably, R¹ is CH₂OH or C(O)NHEt with CH₂OH being most preferred; R², R³, R⁴, R⁵, and R⁶ are each individually selected from the group consisting of hydrogen, halo, CF₃, CN, OR²⁰, C₁₋₄ alkyl, heterocyclyl, and aryl which alkyl and aryl are each optionally substituted with aryl; and R²⁰ is a member selected from the group consisting of H and C₁₋₃ alkyl.

More preferably one subsitutent selected from R², R³, R⁴, R⁵, and R⁶ is a heterocyclyl that is a fused five to seven membered ring containing 1 to 3 heteroatoms selected from O, N, and S. Even more preferably, the heteroatoms are two oxygen atoms, and most preferably, the heteroatoms are two oxygen atoms with points of attachment at the 2 and 3 position.

In a further preferred embodiment, at least one, more preferably two, and even more preferably 3 to 4 of the substituents selected from the group consisting of R², R³, R⁴, R⁵, and R⁶ are hydrogen.

In still a further preferred embodiment, the compounds of this invention are selected from (4S,2R,3R,5R)-2-{6-amino-2-[3-(2-phenylphenoxy)prop-1-ynyl]purin-9-yl}-5-(hydroxymethyl)oxolane-3,4-diol, (4S,2R,3R,5R)-2-[6-amino-2-(3-phenoxyprop-1-ynyl)purin-9-yl]-5-(hydroxymethyl)oxolane-3,4-diol, 4-(3-{9-[(4S,2R,3R,5R)-3,4-dihydroxy-5-(hydroxymethyl)oxolan-2-yl]-6-aminopurin-2-yl}prop-2-ynyloxy)benzenecarbonitrile, (4S,2R,3R,5R)-2-(6-amino-2-[3-(4-phenylphenoxy)prop-1-ynyl]purin-9-yl}-5-(hydroxymethyl)oxolane-3,4-diol, (4S,2R,3R,5R)-2-(6-amino-2-{3-(2-benzylphenoxy]prop-1-ynyl}purin-9-yl)-5-(hydroxymethyl)oxolane-3,4-diol, 2-[2-(3-(2H-benzo[2,3-d]1,3-dioxolen-4-yloxy)prop-1-ynyl)-6-aminopurin-9-yl](4S,2R,3R,5R)-5-(hydroxymethyl)oxolane-3,4-diol, (4S,2R,3R,5R)-2-(6-amino-2-{3-[3,5-bis(tert-butyl)phenoxy]prop-1-ynyl} purin-9-yl)-5-(hydroxymethyl)oxolane-3,4-diol, and mixtures thereof.

The following definitions apply to terms as used herein.
"Halo" or "Halogen" - alone or in combination means all halogens, that is, chloro (Cl), fluoro (F), bromo (Br), iodo (I).
"Hydroxyl" refers to the group -OH.
"Thiol" or "mercapto" refers to the group -SH.
"Alkyl" - alone or in combination means an alkane-derived radical containing from 1 to 20, preferably 1 to 15, carbon atoms (unless specifically defined). It is a straight chain alkyl, branched alkyl or cycloalkyl. Preferably, straight or branched alkyl groups containing from 1-15, more preferably 1 to 8, even more preferably 1-6, yet more preferably 1-4 and most preferably 1-2, carbon atoms, such as methyl, ethyl, propyl, isopropyl, butyl, t-butyl and the like. The term "lower alkyl" is used herein to describe the straight chain alkyl groups described immediately above. Preferably, cycloalkyl groups are monocyclic, bicyclic or tricyclic ring systems of 3-8, more preferably 3-6, ring members per ring, such as cyclopropyl, cyclopentyl, cyclohexyl, adamantyl and the like. Alkyl also includes a straight chain or branched alkyl group that contains or is interrupted by a cycloalkyl portion. The straight chain or branched alkyl group is attached at any available point to produce a stable compound. Examples of this include, but are not limited to, 4-(isopropyl)-cyclohexylethyl or 2-methyl-cyclopropylpentyl. A substituted alkyl is a straight chain alkyl, branched alkyl, or cycloalkyl group defined previously, independently substituted with 1 to 3 groups or substituents of halo, hydroxy, alkoxy, alkylthio, alkylsulfinyl, alkylsulfonyl, acyloxy, aryloxy, heteroaryloxy, amino optionally mono- or di-substituted with alkyl, aryl or heteroaryl groups, amidino, urea optionally substituted with alkyl, aryl, heteroaryl or heterocyclyl groups, aminosulfonyl optionally N-mono- or N,N-di-substituted with alkyl, aryl or heteroaryl groups, alkylsulfonylamino, arylsulfonylamino, heteroarylsulfonylamino, alkylcarbonylamino, arylcarbonylamino, heteroarylcarbonylamino, or the like.
"Alkenyl" - alone or in combination means a straight, branched, or cyclic hydrocarbon containing 2-20, preferably 2-17, more preferably 2-10, even more preferably 2-8, most preferably 2-4, carbon atoms and at least one, preferably 1-3, more preferably 1-2, most preferably one, carbon to carbon double bond. In the case of a cycloalkyl group, conjugation of more than one carbon to carbon double bond is not such as to confer aromaticity to the ring. Carbon to carbon double bonds may be either contained within a cycloalkyl portion, with the exception of cyclopropyl, or within a straight chain or branched portion. Examples of alkenyl groups include ethenyl, propenyl, isopropenyl, butenyl, cyclohexenyl, cyclohexenylalkyl and the like. A substituted alkenyl is the straight chain alkenyl, branched alkenyl or cycloalkenyl group defined previously, independently substituted with 1 to 3 groups or substituents of halo, hydroxy, alkoxy, alkylthio, alkylsulfinyl, alkylsulfonyl, acyloxy, aryloxy, heteroaryloxy, amino optionally mono- or di-substituted with alkyl, aryl or heteroaryl groups, amidino, urea optionally substituted with alkyl, aryl, heteroaryl or heterocyclyl groups, aminosulfonyl optionally N-mono- or N,N-di-substituted with alkyl, aryl or heteroaryl groups, alkylsulfonylamino, arylsulfonylamino, heteroarylsulfonylamino, alkylcarbonylamino, arylcarbonylamino, heteroarylcarbonylamino, carboxy, alkoxycarbonyl, aryloxycarbonyl, heteroaryloxycarbonyl, or the like attached at any available point to produce a stable compound.
"Alkynyl" - alone or in combination means a straight or branched hydrocarbon containing 2-20, preferably 2-17, more preferably 2-10, even more preferably 2-8, most preferably 2-4, carbon atoms containing at least one, preferably one, carbon to carbon triple bond. Examples of alkynyl groups include ethynyl, propynyl, butynyl and the like. A substituted alkynyl refers to the straight chain alkynyl or branched alkenyl defined previously, independently substituted with 1 to 3 groups or substituents of halo, hydroxy, alkoxy, alkylthio, alkylsulfinyl, alkylsulfonyl, acyloxy, aryloxy, heteroaryloxy, amino optionally mono- or di-substituted with alkyl, aryl or heteroaryl groups, amidino, urea optionally substituted with alkyl, aryl, heteroaryl or heterocyclyl groups, aminosulfonyl optionally N-mono- or N,N-di-substituted with alkyl, aryl or heteroaryl groups, alkylsulfonylamino, arylsulfonylamino, heteroarylsulfonylamino, alkylcarbonylamino, arylcarbonylamino, heteroarylcarbonylamino, or the like attached at any available point to produce a stable compound.
"Alkyl alkenyl" refers to a group -R-CR'=CR''' R'''', where R is lower alkyl, or substituted lower alkyl, R', R'", R'''' may independently be hydrogen, halogen, lower alkyl, substituted lower alkyl, acyl, aryl, substituted aryl, hetaryl, or substituted hetaryl as defined below.
"Alkyl alkynyl" refers to a groups -RC≡CR' where R is lower alkyl or substituted lower alkyl, R' is hydrogen, lower alkyl, substituted lower alkyl, acyl, aryl, substituted aryl, hetaryl, or substituted hetaryl as defined below.
"Alkoxy" denotes the group -OR, where R is lower alkyl, substituted lower alkyl, acyl, aryl, substituted aryl, aralkyl, substituted aralkyl, heteroalkyl, heteroarylalkyl, cycloalkyl, substituted cycloalkyl, cycloheteroalkyl, or substituted cycloheteroalkyl as defined.
"Alkylthio" denotes the group -SR, -S(O)ₙ₌₁₋₂-R, where R is lower alkyl, substituted lower alkyl, aryl, substituted aryl, aralkyl or substituted aralkyl as defined herein.
"Acyl" denotes groups -C(O)R, where R is hydrogen, lower alkyl substituted lower alkyl, aryl, substituted aryl and the like as defined herein.
"Aryloxy" denotes groups -OAr, where Ar is an aryl, substituted aryl, heteroaryl, or substituted heteroaryl group as defined herein.
"Amino" denotes the group NRR', where R and R' may independently by hydrogen, lower alkyl, substituted lower alkyl, aryl, substituted aryl, hetaryl, or substituted hetaryl as defined herein or acyL
"Amido" denotes the group -C(O)NRR', where R and R' may independently by hydrogen, lower alkyl, substituted lower alkyl, aryl, substituted aryl, hetaryl, substituted hetaryl as defined herein.
"Carboxyl" denotes the group -C(O)OR, where R is hydrogen, lower alkyl, substituted lower alkyl, aryl, substituted aryl, hetaryl, and substituted hetaryl as defined herein.
"Aryl" - alone or in combination means phenyl or naphthyl optionally carbocyclic fused with a cycloalkyl of preferably 5-7, more preferably 5-6, ring members and/or optionally substituted with 1 to 3 groups or substituents of halo, hydroxy, alkoxy, alkylthio, alkylsulfinyl, alkylsulfonyl, acyloxy, aryloxy, heteroaryloxy, amino optionally mono- or di-substituted with alkyl, aryl or heteroaryl groups, amidino, urea optionally substituted with alkyl, aryl, heteroaryl or heterocyclyl groups, aminosulfonyl optionally N-mono- or N,N-di-substituted with alkyl, aryl or heteroaryl groups, alkylsulfonylamino, arylsulfonylamino, heteroarylsulfonylamino, alkylcarbonylamino, arylcarbonylamino, heteroarylcarbonylamino, or the like.
"Substituted aryl" refers to aryl optionally substituted with one or more functional groups, *e.g*., halogen, lower alkyl, lower alkoxy, alkylthio, acetylene, amino, amido, carboxyl, hydroxyl, aryl, aryloxy, heterocycle, hetaryl, substituted hetaryl, nitro, cyano, thiol, sulfamido and the like.
"Heterocycle" refers to a saturated, unsaturated, or aromatic carbocyclic group having a single ring (*e.g*., morpholino, pyridyl or furyl) or multiple condensed rings (*e.g.,* naphthpyridyl, quinoxalyl, quinolinyl, indolizinyl or benzo[b]thienyl) and having at least one hetero atom, such as N, O or S, within the ring, which can optionally be unsubstituted or substituted with, *e.g*., halogen, lower alkyl, lower alkoxy, alkylthio, acetylene, amino, amido, carboxyl, hydroxyl, aryl, aryloxy, heterocycle, hetaryl, substituted hetaryl, nitro, cyano, thiol, sulfamido and the like.
"Heteroaryl" - alone or in combination means a monocyclic aromatic ring structure containing 5 or 6 ring atoms, or a bicyclic aromatic group having 8 to 10 atoms, containing one or more, preferably 1-4, more preferably 1-3, even more preferably 1-2, heteroatoms independently selected from the group O, S, and N, and optionally substituted with 1 to 3 groups or substituents of halo, hydroxy, alkoxy, alkylthio, alkylsulfinyl, alkylsulfonyl, acyloxy, aryloxy, heteroaryloxy, amino optionally mono- or di-substituted with alkyl, aryl or heteroaryl groups, amidino, urea optionally substituted with alkyl, aryl, heteroaryl or heterocyclyl groups, aminosulfonyl optionally N-mono- or N,N-di-substituted with alkyl, aryl or heteroaryl groups, alkylsulfonylamino, arylsulfonylamino, heteroarylsulfonylamino, alkylcarbonylamino, arylcarbonylamino, heteroarylcarbonylamino, or the like. Heteroaryl is also intended to include oxidized S or N, such as sulfinyl, sulfonyl and N-oxide of a tertiary ring nitrogen. A carbon or nitrogen atom is the point of attachment of the heteroaryl ring structure such that a stable aromatic ring is retained. Examples of heteroaryl groups are pyridinyl, pyridazinyl, pyrazinyl, quinazolinyl, purinyl, indolyl, quinolinyl, pyrimidinyl, pyrrolyl, oxazolyl, thiazolyl, thienyl, isoxazolyl, oxathiadiazolyl, isothiazolyl, tetrazolyl, imidazolyl, triazinyl, furanyl, benzofuryl, indolyl and the like. A substituted heteroaryl contains a substituent attached at an available carbon or nitrogen to produce a stable compound.
"Heterocyclyl" - alone or in combination means a non-aromatic cycloalkyl group having from 5 to 10 atoms in which from 1 to 3 carbon atoms in the ring are replaced by heteroatoms of O, S or N, and are optionally benzo fused or fused heteroaryl of 5-6 ring members and/or are optionally substituted as in the case of cycloalkyl. Heterocycyl is also intended to include oxidized S or N, such as sulfinyl, sulfonyl and N-oxide of a tertiary ring nitrogen. The point of attachment is at a carbon or nitrogen atom. Examples of heterocyclyl groups are tetrahydrofuranyl, dihydropyridinyl, piperidinyl, pyrrolidinyl, piperazinyl, dihydrobenzofuryl, dihydroindolyl, and the like. A substituted hetercyclyl contains a substituent nitrogen attached at an available carbon or nitrogen to produce a stable compound.
"Substituted heteroaryl" refers to a heterocycle optionally mono or poly substituted with one or more functional groups, *e.g*., halogen, lower alkyl, lower alkoxy, alkylthio, acetylene, amino, amido, carboxyl, hydroxyl, aryl, aryloxy, heterocycle, substituted heterocycle, hetaryl, substituted hetaryl, nitro, cyano, thiol, sulfamido and the like.
"Aralkyl" refers to the group -R-Ar where Ar is an aryl group and R is lower alkyl or substituted lower alkyl group. Aryl groups can optionally be unsubstituted or substituted with, *e.g*., halogen, lower alkyl, alkoxy, alkylthio, acetylene, amino, amido, carboxyl, hydroxyl, aryl, aryloxy, heterocycle, substituted heterocycle, hetaryl, substituted hetaryl, nitro, cyano, thiol, sulfamido and the like.
"Heteroalkyl" refers to the group -R-Het where Het is a heterocycle group and R is a lower alkyl group. Heteroalkyl groups can optionally be unsubstituted or substituted with *e.g*., halogen, lower alkyl, lower alkoxy, alkylthio, acetylene, amino, amido, carboxyl, aryl, aryloxy, heterocycle, substituted heterocycle, hetaryl, substituted hetaryl, nitro, cyano, thiol, sulfamido and the like.
"Heteroarylalkyl" refers to the group -R-HetAr where HetAr is an heteroaryl group and R lower alkyl or substituted lower alkyl. Heteroarylalkyl groups can optionally be unsubstituted or substituted with, *e.g.*, halogen, lower alkyl, substituted lower alkyl, alkoxy, alkylthio, acetylene, aryl, aryloxy, heterocycle, substituted heterocycle, hetaryl, substituted hetaryl, nitro, cyano, thiol, sulfamido and the like.
"Cycloalkyl" refers to a divalent cyclic or polycyclic alkyl group containing 3 to 15 carbon atoms.
"Substituted cycloalkyl" refers to a cycloalkyl group comprising one or more substituents with, *e.g*., halogen, lower alkyl, substituted lower alkyl, alkoxy, alkylthio, acetylene, aryl, aryloxy, heterocycle, substituted heterocycle, hetaryl, substituted hetaryl, nitro, cyano, thiol, sulfamido and the like.
"Cycloheteroalkyl" refers to a cycloalkyl group wherein one or more of the ring carbon atoms is replaced with a heteroatom (*e.g*., N, O, S or P).
"Substituted cycloheteroalkyl" refers to a cycloheteroalkyl group as herein defined which contains one or more substituents, such as halogen, lower alkyl, lower alkoxy, alkylthio, acetylene, amino, amido, carboxyl, hydroxyl, aryl, aryloxy, heterocycle, substituted heterocycle, hetaryl, substituted hetaryl, nitro, cyano, thiol, sulfamido and the like,
"Alkyl cyc oalkyl" denotes the group -R-cycloalkyl where cycloalkyl is a cycloalkyl group and R is a lower alkyl or substituted lower alkyl. Cycloalkyl groups can optionally be unsubstituted or substituted with *e.g*. halogen, lower alkyl, lower alkoxy, alkylthio, acetylene, amino, amido, carboxyl, hydroxyl, aryl, aryloxy, heterocycle, substituted heterocycle, hetaryl, substituted hetaryl, nitro, cyano, thiol, sulfamido and the like.
"Alkyl cycloheteroalkyl" denotes the group -R-cycloheteroalkyl where R is a lower alkyl or substituted lower alkyl. Cycloheteroalkyl groups can optionally be unsubstituted or substituted with *e.g.* halogen, lower alkyl, lower alkoxy, alkylthio, amino, amido, carboxyl, acetylene, hydroxyl, aryl, aryloxy, heterocycle, substituted heterocycle, hetaryl, substituted hetaryl, nitro, cyano, thiol, sulfamido and the like.

The compounds of this invention can be prepared as outlined in Schemes 1-3 below.

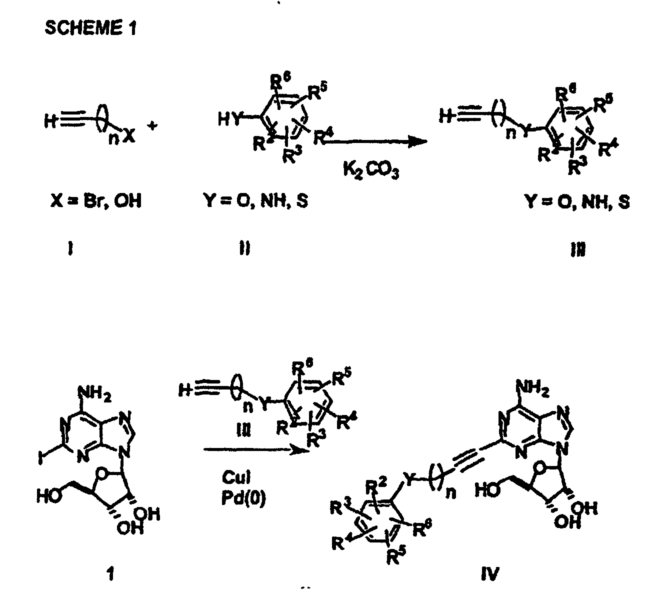

Compounds having the general formula IV (Scheme 1) can be prepared by the palladium mediated cross-coupling of compound 1 with alkynyl derivatives (Y= O, NH, S) represented by the formula III (Scheme 1) in the presence or absence of copper salts (Matsuda et al, Chem. Phar. Bull, (1985), 33, 19766-19769; Kochetkov et al., Organic Chemistry of Nucleic Acids, Part B, Plenum Press, New York, (1972), 333; Nair, et al., Tet. Lett. (1990), 31, 807-810). In some cases compound III can be obtained from commercial sources. Examples of commercially available Compound III compounds include phenyl propargyl ether, 3-(2,6-dichlorophenoxy)prop-1-yne, 3-(4-cyanophenoxy)prop-1-yne, 3-(4-carboxyethyl-phenoxy)prop-1-yne, 3-(4-cyanophenoxy)-3-methyl-but-1-yne, and phenyl propargyl sulfide. Compound III can be prepared either by the reaction of propargyl or homopropargyl bromide with substituted phenols, thiophenols and anilines using a base (*e.g*. potassium carbonate) in an appropriate solvent (*e.g.* DMF, acetone). Specifically, when *Y* = NH, the aniline starting material can be activated by making a trifluoroacetamide followed by alkylation. An alternative synthesis of compound III can be accomplished by reacting propargyl or homopropargyl alcohols with phenols and thiophenols under Mitsunobu reaction conditions (O. Mitsunobu, Bull. Chem. Soc. Jpn., (1967), 40, 2380; A. Hassner, J. Org. Chem. (1990), 55, 2243).

Compounds with general formula VII can be prepared as shown in Scheme 2. 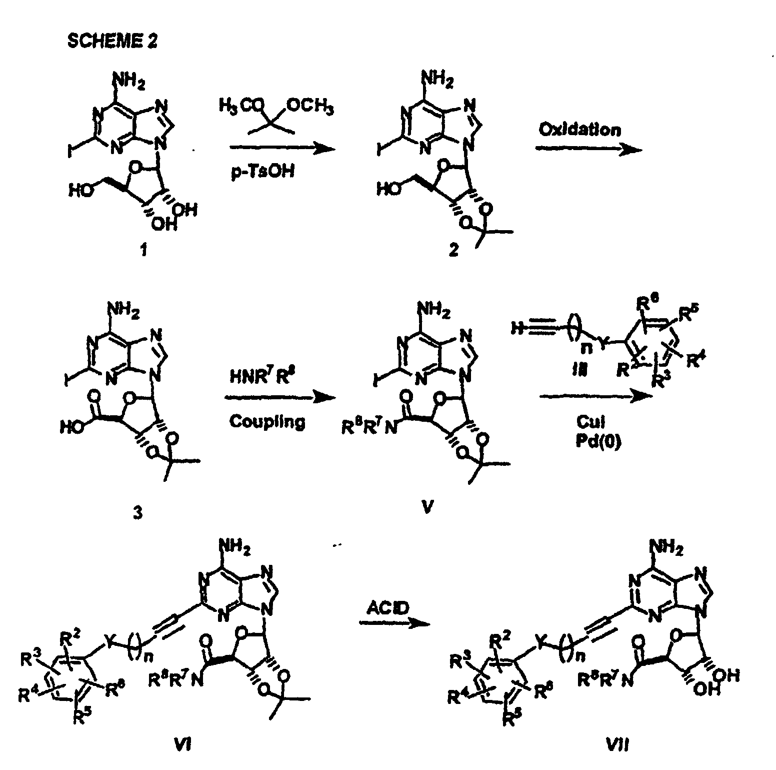 Compound 2, which can be obtained by reacting compound 1 with 2,2-dimethoxypropane in the presence of an acid, can be oxidized to the carboxylic acid 3, based on structurally similar compounds, using potassium permanganate or pyridinium chlorochromate, or TEMPO etc., (M. Hudlicky, (1990) Oxidations in Organic Chemistry, ACS Monographs, American Chemical Society, Washington D. C.; B. Cox et al, WO 9828319) to compound 3. Reaction of a primary or secondary amine with the formula HNR⁶R⁷, and compound 3 using DCC (M. Fujino et al., Chem. Pharm. Bull. (1974), 22, 1857), PyBOP (J. Martinez et al., J. Med. Chem. (1988) 28, 1874) or PyBrop (J. Caste et al. Tetrahedron, (1991), 32, 1967) coupling conditions can afford compound V. Compound VI can be prepared by the palladium mediated cross-coupling of compound V with alkynyl derivatives (Y= O, N, S) represented by the formula III (Scheme 1), in an appropriate solvent (*e.g*. DMF, acetone) in the presence or absence of copper salts. Deprotection of compound VI can be performed by heating with 80% aqueous acetic acid (T. W. Greene and P. G. M. Wuts, (1991) Protective Groups in Organic Synthesis, A. Whey-Interscience publication) or with anhydrous hydrochloric acid (4N) to obtain compound VII.

A specific synthesis of compound 10 is illustrated in Scheme 3. 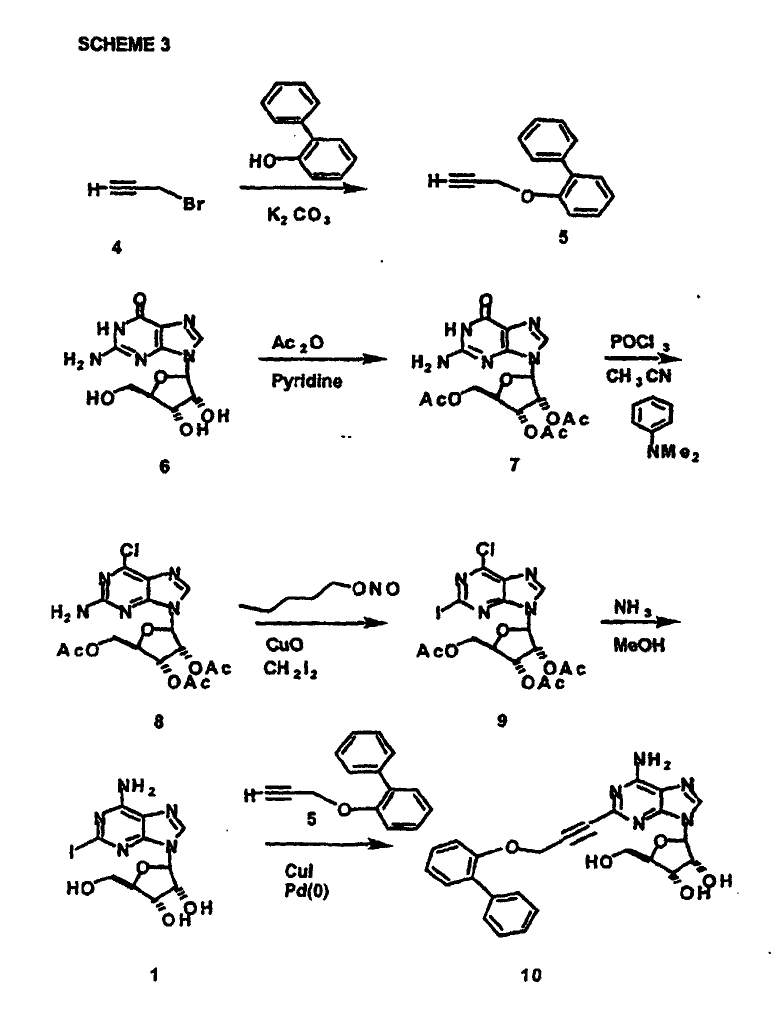 Commercially available guanosine (compound 6) was converted to the triacetate 7 as previously described (M. J. Robins and B. Uznanski, Can. J. Chem. (1981), 59, 2601-2607). Compound 8, prepared by following the literature procedure of Cerster et al. (J. F. Cerster, A. F. Lewis, and R. K. Robins, Org. Synthesis, 242-243), was converted to compound 1 in two steps as previously described (V. Nair et al., J. Org. Chem., (1988), 53, 3051-3057). Compound 1 was subjected to palladium mediated cross-coupling, utilizing dichloro-bis-(triphenylphosphine) palladium (II) as a catalyst, with compound 5 to provide compound 10. Compounds 11-18 were prepared in a similar manner.

The methods used to prepare the compounds of this invention are not limited to those described above. Additional methods can be found in the following sources: J. March, Advanced Organic Chemistry; Reaction Mechanisms and Studies (1992), A Wiley Interscience Publications and J. Tsuji, Palladium Reagents and Catalyst, Innovations in Organic Synthesis (1996), John Wiley & Sons Publications.

Compounds of this invention are useful in conjunction with radioactive imaging agents to image coronary activity. The compounds of this invention are A_{2A} agonists that are believed to provide specific activation of adenosine A_{2A} receptors in the coronary vessels as opposed to adenosine A₁ receptors in the atrium and AV-node and/or A_{2B} receptors in peripheral vessels, thus avoiding undesirable side-effects. Upon administration in a therapeutic amount, the compounds of this invention cause coronary blood vessels to vasodilate to induce coronary steal wherein healthy coronary vessels steal blood from unhealthy vessels resulting in lack of blood flow to heart tissues. Coronary imaging then identified coronary regions with healthy and unhealthy blood flow. Lower doses of the A_{2A} agonists may provide beneficial coronary vasodilatation (less severe) in the treatment of chronic CAD.

As A_{2A} agonists, the compounds of this invention are also useful in adjunctive therapy with angioplasty to induce dilation, inhibit platelet aggregation, arid as a general anti-inflammatory agent. A_{2A} agonists, such as the compounds of this invention, can provide the therapeutic benefits described above by preventing neutrophil activation (Purinergic Approaches in Experimental Therapeutics K. A. Jacobson and M. F. Jarvis 1997 Wiley, New York). The compounds of this invention are also effective against a condition called no-reflow in which platelets and neutrophils aggregate and block a vessel. As A_{2A} agonists, the compounds of this invention are effective against no-reflow by preventing neutrophil and platelet activation (e.g., they are believed to prevent release of superoxide from neutrophils). As A_{2A} agonists, the compounds of this invention are also useful as cardioprotective agents through their anti-inflammatory action on neutrophils. Thus, in situations when the heart will go through an ischemic state such as a transplant, they will be useful.

This invention also includes pro-drugs of the above-identified A_{2A} agonists. A pro-drug is a drug that has been chemically modified and may be biological inactive at its site of action, but which will be degraded or modified by one or more enzymatic or *in vivo* processes to the bioactive form. The pro-drugs of this invention should have a different pharmacokinetic profile to the parent enabling improved absorption across the mucosal epithelium, better salt formulation and/or solubility and improved systemic stability. The above-identified compounds may be preferably modified at one or more of the hydroxyl groups. The modifications may be (1) ester or carbamate derivatives which may be cleaved by esterases or lipases, for example; (2) peptides which may be recognized by specific or non-specific proteinase; or (3) derivatives that accumulate at a site of action through membrane selection or a pro-drug form or modified pro-drug form, or any combination of (1) to (3) above.

The compositions may be administered orally, intravenously, through the epidermis or by any other means known in the art for administering a therapeutic agents. The method of treatment comprises the administration of an effective quantity of the chosen compound, preferably dispersed in a pharmaceutical carrier. Dosage units of the active ingredient are generally selected from the range of 0.01 to 100 mg/kg, but will be readily determined by one skilled in the art depending upon the route of administration, age and condition of the patient. This dose is typically administered in a solution about 5 minutes to about an hour or more prior to coronary imaging. No unacceptable toxicological effects are expected when compounds of the invention are administered in therapeutic amounts.

If the final compound of this invention contains a basic group, an acid addition salt may be prepared. Acid addition salts of the compounds are prepared in a standard manner in a suitable solvent from the parent compound and an excess of acid, such as hydrochloric, hydrobromic, sulfuric, phosphoric, acetic, maleic, succinic, or methanesulfonic. The hydrochloric salt form is especially useful. If the final compound contains an acidic group, cationic salts may be prepared. Typically the parent compound is treated with an excess of an alkaline reagent, such as hydroxide, carbonate or alkoxide, containing the appropriate cation. Cations such as Na⁺, K⁺, Ca⁺² and NH₄⁺ are examples of cations present in pharmaceutically acceptable salts. Certain of the compounds form inner salts or zwitterions which may also be acceptable.

Pharmaceutical compositions including the compounds of this invention, and/or derivatives thereof, may be formulated as solutions or lyophilized powders for parenteral administration. Powders may be reconstituted by addition of a suitable diluent or other pharmaceutically acceptable carrier prior to use. If used in liquid form the compositions of this invention are preferably incorporated into a buffered, isotonic, aqueous solution. Examples of suitable diluents are normal isotonic saline solution, standard 5% dextrose in water and buffered sodium or ammonium acetate solution. Such liquid formulations are suitable for parenteral administration, but may also be used for oral administration. It may be desirable to add excipients such as polyvinylpyrrolidinone, gelatin, hydroxycellulose, acacia, polyethylene glycol, mannitol, sodium chloride, sodium citrate or any other excipient known to one of skill in the art to pharmaceutical compositions including compounds of this invention. Alternatively, the pharmaceutical compounds may be encapsulated, tableted or prepared in an emulsion or syrup for oral administration. Pharmaceutically acceptable solid or liquid carriers may be added to enhance or stabilize the composition, or to facilitate preparation of the composition. Liquid carriers include syrup, peanut oil, olive oil, glycerin, saline, alcohols and water. Solid carriers include starch, lactose, calcium sulfate, dihydrate, teffa alba, magnesium stearate or stearic acid, talc, pectin, acacia, agar or gelatin. The carrier may also include a sustained release material such as glycerol monostearate or glycerol distearate, alone or with a wax. The amount of solid carrier varies but, preferably, will be between about 20 mg to about 1 gram per dosage unit The pharmaceutical dosages are made using conventional techniques such as milling, mixing, granulation, and compressing, when necessary, for tablet forms; or milling, mixing and filling for hard gelatin capsule forms. When a liquid carrier is used, the preparation will be in the form of a syrup, elixir, emulsion or an aqueous or non-aqueous suspension. Such a liquid formulation may be administered directly or filled into a soft gelatin capsule. It is preferred that the compositions of this invention are administered as a solution either orally or intravenously by continuos infusion or bolus.

The Examples which follow serve to illustrate this invention. The Examples are intended to in no way limit the scope of this invention, but are provided to show how to make and use the compounds of this invention. In the Examples, all temperatures are in degrees Centigrade.

### EXAMPLE 1

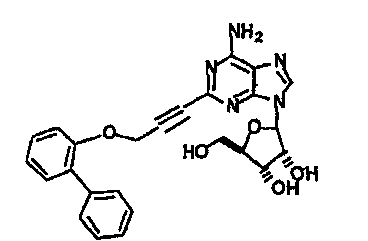

**(4S,2R,3R,5R)-2-{6-amino-2-[3-(2-phenylphenoxy)prop-1-ynyl]purin-9-yl}-5-(hydroxymethyl)oxolane-3,4-diol (10).**

Synthesis of 2-phenyl-1-prop-2-ynyloxybenzene (compound 5). 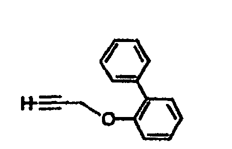 To a solution of propargyl bromide (80% solution in toluene, 500 δ 3.36 mmol) in acetone (15 ml) at 23 °C was added 2-phenyl phenol (316 mg, 1.86 mmol) and potassium carbonate (1.05 g, 7.61 mmol). After being stirred in a sealed reaction vial at 65°C for 14 hours, the reaction was concentrated *in vacuo*, the residue purified by flash chromatography (ethyl acetate: hexane: 9:1) to afford compound 5 in 95% yield. ¹H NMR(CDCl₃) δ 2.45-2.55 (m, 1H), 4.60-4.70 (m, 2H), 6.90-7.43 (m, 9H).

### Synthesis of (4S,2R,3R,5R)-2-{6-amino-2-[3-(2-phenylphenoxy)prop-1-ynyl]purin-9-yl}-5-(hydroxymethyl)oxolane-3,4-diol (compound 10).

To a solution of (4S,2R,3R,5R)-2-(6-amino-2-iodopurin-9-yl)-5-(hydroxymethyl)oxolane-3,4-diol (compound 1)(50 mg, 0.126 mmol) and prop-2-ynyloxybenzene (22 mg, 0.1605 mmol) in N, N-dimethylformamide (1 ml) and triethylamine (21 mg, 16.065mmol) at 23°C was added copper iodide (5mg, 0.026 mmol) and dichlorobis(triphenylphosphine)palladium(II) (22 mg, 0.031 mmol) catalyst. After being stirred in a sealed reacti-vial at 80 C for 6 hours, the reaction was concentrated *in vacuo*, the residue purified by preparatory thin layer chromatography (methylene chloride: methanol 9:1) to afford compound 10 (9.6 mg, 0.024 mmol) in 20% yield. ¹H NMR(CDCl₃:CD₃OD 9:1) δ 3.02-3.04 (m, 1H), 3.07 (s, 1H), 3.55, 3.74 (dd, 2H), 4.02 (s, 1H), 4.11-4.13 (m, 1H), 4.48-4.52 (m, 1H), 4.72 (s, 2H), 5.65 (d, 1H), 6.75-6.82 (m, 3H), 7.08-7.12 (m, 2H), 7.94 (s, 1H).

### EXAMPLE 2

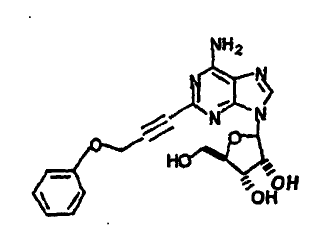

**(4S,2R,3R,5R)-2-[6-amino-2-(3-phenoxyprop-1-ynyl)purin-9-yl]-5-(hydroxymethyl)oxolane-3,4-diol (11)**

Compound 11 was prepared in the manner of compound 10. ¹H NMR (CDCl₃:CD₃OD 9:1) δ3.02-3.04 (m, 1H), 3.07 (s, 1H), 3.55, 3.74 (dd, 2H), 4.02 (s, 1H), 4.11- 4.13 (m, 1H), 4.48-4.52 (m, 1H), 4.72 (s, 2H), 5.65 (d, 1H), 6.75-6.82 (m, 3H), 7.08-7.12 (m, 2H), 7.94 (s, 1H). ), 4.48-4.52 (m, 1H), 4.72 (s, 2H), 5.65 (d, 1H), 6.90-7.00 (m, 1H), 7.10-7.15 (m, 1H), 7.15-7.32 (m, 5H), 7.35-7.45 (m, 2H), 7.94 (s, 1H).

### EXAMPLE 3

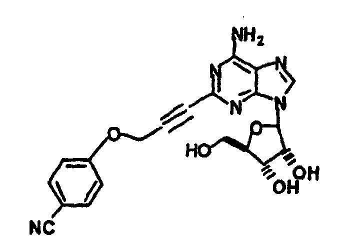

**4-(3-{9-[(4S,2R,3R,5R)-3,4-dihydroxy-5-(hydroxymethyl)oxolan-2-yl]-6-aminopurin-2-yl} prop-2-ynyloxy)benzenecarbonitrile (12)**

Compound 12 was prepared in the manner of compound 10. ¹H NMR(CDCl₃:CD₃OD 9:1) δ3.55 (d, 1H), 3.70 (d, 1H), 4.05-4.07 (m, 1H), 4.10-4.12 (m, 1H), 4.48 (dd, 1H), 4.80 (s, 2H), 5.65 (d, 1H), 6.90 (d, 2H), 7.45 (d, 2H), 7.95 (s, 1H)

### EXAMPLE 4

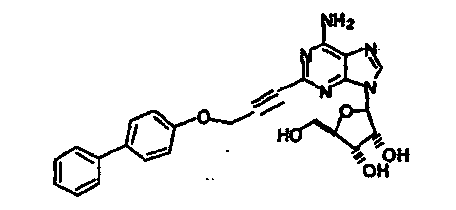

**(4S,2R,3R,5R)-2-{6-amino-2-[3-(4-phenylphenoxy)prop-1-ynyl]purin-9-yl}-5-(hydroxymethyl)oxolane-3,4-diol (13)**

Compound 13 was prepared in the manner of compound 10. ¹H NMR(CDCl₃:CD₃OD 9:1) δ3.02-3.04 (m, 1H), 3.07 (s, 1H), 3.55, 3.74 (dd, 2H), 4.02 (s, 1H), 4.11- 4.13 (m, 1H), 4.48-4.52 (m, 1H), 4.72 (s, 2H), 5.65 (d, 1H), 6.90-7.00 (m, 1H), 7.10-7.15 (m, 1H), 7.15-7.32 (m, 5H), 7.35-7.45 (m, 2H), 7.94 (s, 1H).

### EXAMPLE 5

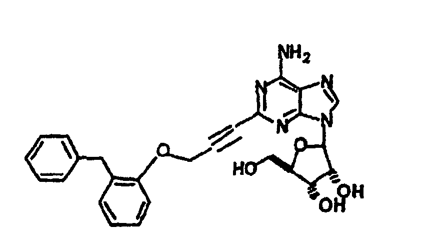

**(4S,2R,3R,5R)-2-(6-amino-2-{3-[2-benzylphenoxy]prop-1-ynyl} purin-9-yl)-5-(hydroxymethyl)oxolane-3,4-diol (14)**

Compound 14 was prepared in the manner of compound 10. ¹H NMR(CDCl₃:CD₃OD 9:1) δ3.02-3.04 (m, 1H), 3.07 (s, 1H), 3.55, 3.74 (dd, 2H), 3.92 (s, 2H), 4.02 (s, 1H), 4.11-4.13 (m, 1H), 4.48-4.52 (m, 1H), 4.72 (s, 2H), 5.65 (d, 1H), 6.80-6.88 (m, 1H), 6.96-7.01 (m, 2H), 7.15-7.22 (m, 6H), 7.94(s, 1H).

### EXAMPLE 6

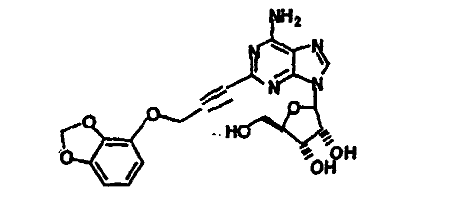

**2-[2-(3-(2H-benzo[2,3-d]1,3-dioxolen-4-yloxy)prop-1-ynyl)-6-aminoparin-9-yl](4S,2R,3R,5R)-5-(hydroxymethyl)oxolane-3,4-diol (15).**

Compound 15 was prepared in the manner of compound 10. ¹H NMR(CDCl₃:CD₃OD 9:1)_3.61 (d, J = 13.2 Hz, 1H), 3.78 (d, J = 13.2 Hz, 1H) 4.10-4.12 (m, 1H), 4.21-4.22 (m, 1H), 4.58-4.59 (m, 1H), 4.68 (s, 2H, O-CH2-O), 5.73-5.75 (m, 3H), 6.30 (d, J=8.4Hz, 2H), 6.45 (s,1H), 6.54 (d, J=8.4 Hz, 2H), 8.07 (s, 1H).

### EXAMPLE 7

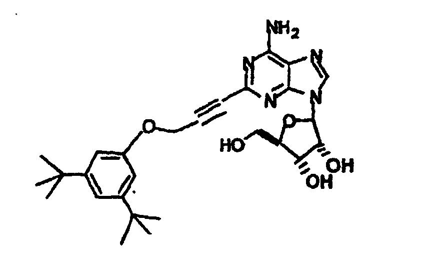

**(4S,2R,3R,5R)-2-(6-amino-2-{3-[3,5-bis(tert-butyl)phenoxy]prop-1-ynyl} purin-9-yl)-5-(hydroxymethyl)oxolane-3,4-diol (16).**

Compound 16 was prepared in the manner of compound 10. ¹H NMR(CDCl₃:CD₃OD 9:1) δ1.22 (s, 9H), 3.02-3.04 (m, 1H), 3.07 (s, 1H), 3.55, 3.74 (dd, 2H), 4.02 (s, 1H), 4.11-4.13 (m, 1H), 4.48-4.52 (m, 1H), 4.72 (s, 2H), 5.65 (d, 1H), 6.78 (s, 2H), 6.99 (s, 1H), 7.94 (s, 1H).

### EXAMPLE 8

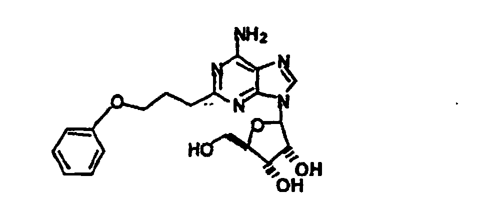

**(4S,2R,3R,5R)-2-[6-amino-2-(3-phenoxypropyl)purin-9-yl]-5-(hydroxymethyl)oxolane-3,4-diol (17).**

Compound 17 was prepared in the manner of compound 10. ¹H NMR(CDCl₃:CD₃OD 9:1) δ2.15-2.25(m, 2H), 3.02-3.04 (m, 1H), 3.07 (s, 1H), 3.55, 3.74 (dd, 2H), 3.90-4.00 (m, 2H), 4.02 (s, 1H), 4.11- 4.13 (m, 1H), 4.48-4.52 (m, 1H), 4.65-4.75 (s, 2H), 5.65 (d, 1H), 6.75-6.82 (m, 3H), 7.08-7.12 (m, 2H), 7.94 (s, 1H).

### EXAMPLE 9

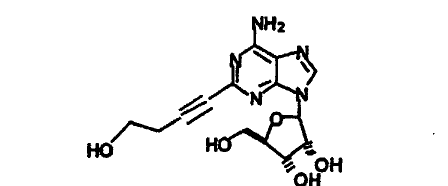

**(4S,2R,3R,5R)-2-[6-amino-2-(4-hydroxybut-1-ynyl)purin-9-yl]-5-(hydroxymethyl)oxolane-3,4-diol(18)**

Compound 18 was prepared in the manner of compound 10. ¹H NMR(CDCl₃:CD₃OD 9:1) δ2.45-2.55 (m, 2H), 3.02-3.04 (m, 1H), 3.07 (s, 1H), 3.55, 3.74 (dd, 2H), 4.02 (s, 1H), 4.11- 4.13 (m, 1H), 4.48-4.52 (m, 1H), 4.65-4.73 (m, 2H), 5.65 (d, 1H), 7.94 (s, 1H).

### EXAMPLE 10

Compounds of this invention were assayed to determine their affinity for the A_{2A} receptor in a pig striatum membrane prep. 0.2 mg of pig striatal membranes were treated with adenosine deaminase and 50 mM Tris buffer (pH = 7.4) followed by mixing. To the pig membranes was added 2 µL of serially diluted DMSO stock solution of the compounds of this invention at concentrations ranging from 100 µM to 10 nM or the control received 2 µL of DMSO alone, then the tritiated antagonist ZM 241385 in Tris buffer (50 mM, pH of 7.4) was added to achieve a final concentration of 2 nM. After incubation at 23°C for 2h, the solutions were filtered using a membrane harvester using multiple washing of the membranes (3 x). The filter disks were counted in scintillation cocktail affording the amount of displacement of tritiated ZM by the competitive binding compounds of this invention. Greater than a 5 point curve was used to generate IC50's and the number of experiments (n) is indicated in the column marked in Table 1 below.

**TABLE 1**

| Compound Number | Ki (nM) | n |
|---|---|---|
| **10** | ++ | 2 |
| **11** | +++ | 4 |
| **12** | ++ | 3 |
| **13** | + | 3 |
| **14** | ++ | 3 |
| **15** | +++ | 1 |
| **16** | + | 2 |
| +++ = 10 - 100 nM | | |
| ++ = 100 - 1,000 nM | | |
| + = 1,000 - 10,000 nM | | |

These results indicate that the compounds of this invention are potent enough to be useful as vasodilators.

The compounds of Tables 2-5 may be prepared using the synthesis schemes described above:

**TABLE 2**

| | | |
|---|---|---|
| R₂, R₃, and R₄ = H; | | |

| **R**_{**1**} | **R**_{**5**} | **R**_{**6**} |
|---|---|---|
| CH₂OH | 3-methoxy | 5-methoxy |
| CH₂OH | 3-ethoxy | 5-ethoxy |
| CH₂OH | 3-propoxy | 5-propoxy |
| CH₂OH | 3-iso-propoxy | 5-iso-propoxy |
| CH₂OH | 3-methyl | 5-methyl |
| CH₂OH | 3-ethyl | 5-ethyl |
| CH₂OH | 3-propyl | 5-propyl |
| CH₂OH | 3-iso propyl | 5-iso propyl |
| CH₂OH | 3-methyl | 5-isopropyl |
| CH₂OH | 3-trifluoromethyl | 5-trifluoromethyl |
| CH₂OH | 3-chloro | 5-chloro |
| CH₂OH | 3-fluoro | 5-fluoro |
| CH₂OH | 2-methoxy | 3-methoxy |
| CH₂OH | 2-methyl | 3-methyl |
| CH₂OH | 3-methoxy | 4-methoxy |

**TABLE 3**

| | |
|---|---|
| R₂, R₃, R₄, and R₅ = H; | |

| **R**_{**1**} | **R**_{**6**} |
|---|---|
| CH₂OH | 3-methyl |
| CH₂OH | 3-ethyl |
| CH₂OH | 3-propyl |
| CH₂OH | 3-iso-propyl |
| CH₂OH | 3-sec-butyl |
| CH₂OH | 3-tent-butyl |
| CH₂OH | 3-trifluoromethyl |
| CH₂OH | 4-tert―butyl |
| CH₂OH | 4-chloro |
| CH₂OH | 4-fluoro |

**TABLE 4**

| | | |
|---|---|---|
| R₂, R₃, and R₄ = H; | | |

| **R**_{**1**} | **R**_{**5**} | **R**_{**6**} |
|---|---|---|
| C(O)NHEt | 3-methoxy | 5-methoxy |
| C(O)NHEt | 3-ethoxy | 5-ethoxy |
| C(O)NHEt | 3-propoxy | 5-propoxy |
| C(O)NHEt | 3-iso-propoxy | 5-iso-propoxy |
| C(O)NHEt | 3-methyl | 5-methyl |
| C(O)NHEt | 3-ethyl | 5-ethyl |
| C(O)NHEt | 3-propyl | 5-propyl |
| C(O)NHEt | 3-isopropyl | 5-iso propyl |
| C(O)NHEt | 3-methyl | 5-isopropyl |
| C(O)NHEt | 3-trifluoromethyl | 5-trifluoromethyl |
| C(O)NHEt | 3-chloro | 5-chloro |
| C(O)NHEt | 3-fluoro | 5-fluoro |
| C(O)NHEt | 2-methoxy | 3-methoxy |
| C(O)NHEt | 2-methyl | 3-methyl |
| C(O)NHEt | 3-methoxy | 4-methoxy |

**TABLE 5**

| | |
|---|---|
| R₂, R₃, R₄, and R₅ = H; | |

| **R**_{**1**} | **R**_{**6**} |
|---|---|
| C(O)NHEt | 3-methyl |
| C(O)NHEt | 3-ethyl |
| C(O)NHEt | 3-propyl |
| C(O)NHEt | 3-iso-propyl |
| C(O)NHEt | 3-sec-butyl |
| C(O)NHEt | 3-tert-butyl |
| C(O)NHEt | 3-trifluoromethyl |
| C(O)NHEt | 4-tert―butyl |
| C(O)NHEt | 4-chloro |
| C(O)NHEt | 4-fluoro |

### EXAMPLE 11

The objective of this experiment was to determine the affinities and receptor binding selectivity of a compound of this invention for A₁, A_{2A}, A_{2B} and A₃ adenosine receptors. Molecular cloning has identified and confirmed the existence of four subtypes of adenosine receptors (AdoRs), designated as A₁, A_{2A}, A_{2B} and A₃AdoRs (Linden, 1994). These AdoR subtypes have distinct anatomical distributions, pharmacological properties and physiological functions (Shryock and Belardinelli, 1997). A₁ and A₃AdoRs couple to inhibitory G proteins (Gᵢ/ₒ) and decrease the activity of adenylyl cyclase, whereas A_{2A} and A_{2B}AdoRs increase intracellular cAMP content via coupling to stimulatory G proteins (Gs).

Ligands with high potency and tissue/organ selectivity for distinct adenosine receptor subtypes have therapeutic and diagnostic potentials for a variety of diseases (such as arrhythmias, ischemic heart diseases, asthma and Parkinson's disease) and are the focus of considerable research efforts by both academia and industry. Here we report the pharmacological and functional characterization of a series of novel adenosine analogues of this invention using mammalian cell lines expressing either endogenous AdoRs or recombinant human AdoRs.

### Materials

Adenosine deaminase was purchased from Boehringer Manheim Biochemicals Indianapolis, IN, U.S.A). [³H]ZM241385 was purchased from Tocris Cookson Ltd (Langford, Bristol, UK). [³H]CPX was from New England Nuclear (Boston, MA, USA). CGS21680, NECA, R-PIA, Rolipram and HEK-hA_{2A}AR membranes were obtained from Sigma-RBI (Natick, MA). WRC-0470 was prepared as described in the literature (K. Niiya et al., J. Med. Chem. 35: 4557-4561 (1992). Compound 11 of this invention was synthesized as described above and prepared as a stock solution (10 mmol/L) in DMSO.

Cell culture and membrane preparation- PC12 cells were obtained from the American Type Culture Collection and grown in DMEM with 5% fetal bovine serum, 10% horse serum, 0.5 mmol/L L-glutamine, 100 U/mL penicillin, 0.1 mg/mL streptomycin, and 2.5 µg/mL amphotericin. HEK-293 cells stably expressing recombinant human A_{2B}AdoRs (HEK-hA_{2B}AdoR) were grown in DMEM supplemented with 10% fetal bovine serum and 0.5 mg/mL G-418. CHOK1 cells stably expressing the recombinant human A₁AdoR (CHO-hA₁AdoR) and A₃AdoR (CHO-hA₃AdoR) were grown as monolayers on 150-mm plastic culture dishes in Ham's F-12 media supplemented with 10% fetal bovine serum in the presence of 0.5 mg/mL G-418. Cells were cultured in an atmosphere of 5% CO₂ / 95% air maintained at 37°C.

To make membranes, cells were detached from the culture plates into ice-cold 50 mmol/L Tris-HCI buffer (pH7.4). The cell suspensions were homogenized with Polytron at setting 4 for 30 seconds, and spun at 48,000g for 15 minutes. The pellets were washed three times by re-suspension in ice-cold Tris-HCI buffer and centrifugation. The final pellet was re-suspended in a small volume of Tris-HCI, aliquoted and frozen at -80°C until used for receptor binding assays. The protein concentration of membrane suspensions was determined using the Bradford method (Bio-Rad) with bovine serum as standards.

**Competition Binding Assays-** Competition assays were performed to determine the affinities (Kᵢ) of the following unlabeled compounds (competing agents): Compounds WRC-0470; Compound 11 of this invention; NECA; CGS 21680; and R-PIA for A₁AdoRs ([³H]DPCPX binding sites on CHO-hA₁AdoR cell membranes), A_{2A}AdoRs([³H]ZM241385 binding sites on PC12 and HEK-hA_{2A}AR cell membranes), A_{2B}AdoR ([³H]DPCPX binding sites on HEK-hA_{2B}AdoR cell membranes) and A₃AdoR ([¹²⁵I]ABMECA binding sites on CHO-hA₃AdoR cell membrane). Membrane suspensions were incubated for 2 hours at room temperature in 50 mmol/L Tris-HCI buffer (pH 7.4) containing ADA (1 U/mL), Gpp(NH)p (100 µM), radioligand {either [³H]ZM241385 (-1.5 to 5 nmol/L), [³H]DPCPX (∼2.5 to 3.0 nmol/L for A, and 30 nM for A_{2B}) or [¹²⁵I]ABMECA (1 nM)} and progressively higher concentrations of the competing agents. At the end of incubation, bound and free radioligands were separated by filtration through Whatman GF/C glass fiber filters using a Brandel tissue harvester (Gaithersburg, MD). Triplicate determinations were performed for each concentration of the competing agent.

### Study Design (Protocols)

The affinity (Kᵢ) of Compound 11 of this invention for the A₁ and A_{2A} adenosine receptor was determined by its potency to compete for [³H]CPX (A,) or [³ H]ZM241385 (A_{2A}) binding sites on membranes derived from CHO-hA₁AdoR, PC12 or HEK-HA_{2A}AdoR cells. R-PIA and CGS21680, agonists that are selective for A₁ and A_{2A} respectively, and NECA, a non-selective AdoR agonist were used as controls. To facilitate comparison and avoid the complication of multiple affinity states due to receptor coupling to G-proteins, the competition binding studies were carried out in the presence of Gpp (NH) p (100 µM) to uncouple receptors from G-proteins. The affinity of selected compounds for A_{2B} and A₃ receptors were assessed by their potencies to compete for [³H] CPX (A_{2B}) and [¹²⁵I] ABMECA (A3) binding sites on membranes derived from HEK-hA_{2B}AdoR and CHO-hA,AdoR cells, respectively.

The functional potency and selectivity of these drugs for A_{2A} vs. A_{2B}AdoRs were assessed by determining their effects on A_{2A} or A_{2B}-mediated cAMP accumulation in PC 12 and HEK-293 cells, respectively. In these experiments, CGS21680 and NECA were used as positive controls.

### Results

The affinity (Kᵢ) of WRC-0470 and Compound 11 of this invention for human A₁, rat and human A_{2A}AdoRs, as determined by competition binding studies are summarized in Table 6, below. All compounds show moderate selectivity for human A_{2A} versus A₁ receptor. Furthermore, Compound 16 (N-Py), at a concentration of 10 µM, decreased the specific binding of [³H] CPX (HEK-hA_{2B}AdoR) or [¹²⁵I]ABMECA (CHO-hA₃AdoR) by 20% and 22%, respectively.

**Table 6.**

| **Binding Affinities of Adenosine Receptor Agonists for A**_{**2A**}**AdoRs and A**_{**1**}**AdoRs K**_{**i**}**/ nmol/L (pK**_{**i**}**±SEM)** | | | | |
|---|---|---|---|---|
| | HEK-hA_{2A}AR Cells | | CHO-hA₁AR | |
| | Binding Affinity | n | Binding Affinity | n |
| Compound 11 | 305 (6.52±0.04) [0.75±0.09] | 6 | 866 (6.07±0.05) [0.87±0.07] | 3 |
| WRC-0470 | 272 (6.55±0.04) [0.83±0.07] | 6 | 7278 (5.16±0.09) [1.13±0.21] | 3 |
| NECA | 360 (6.45±0.06) [0.83±0.08] | 3 | 328 (6.49±0.06) [0.88±0.03] | 3 |
| R-PIA | 1656(5.78±0.02) [1.05±0.02) | 3 | 477(6.35±0.11) [1.03±0.08) | 3 |

The results of this Experiment show that Compound 11 is a high affinity A_{2A} agonist.

### EXAMPLE 12

The objective of this Example was to characterize pharmacologically the effects of A_{2A} AdoR agonists of this invention on coronary artery conductance. Specifically, the experiment was designed to determine the potency of Compound 11 of this invention and to compare its potency with that of adenosine and other selected A_{2A} AdoR agonists,.

In the heart, the A_{2A} adenosine receptor mediates the coronary vasodilation caused by adenosine, whereas the A₁ receptor mediates the cardiac depressant actions of adenosine, such as the negative chronotropic and dromotropic (AV block) effects.

Several potent and selective ligands, both agonists and antagonists, for the A1 and A_{2A} AdoRs have been synthesized. In the heart agonists of A₁ AdoRs have been proposed to be useful as antiarrhymic agents, whereas agonists of A_{2A} AdoRs are being developed for selective coronary vasodilation

A series of adenosine derivatives targeted for selective activation of A_{2A} adenosine receptor (A_{2A} AdoR) were synthesized for the purposes of developing coronary vasodilators. More specifically, in this study we report on the effect of a series of novel A_{2A} AdoR agonists on coronary artery conductance (vasodilation) in rat and guinea pig isolated perfused hearts.

### Materials

Rats (Sprague Dawley) and Guinea pigs (Hartley) were purchased from Simonsen and Charles Rivers, respectively. WRC-0470 was prepared as described in the literature (K. Niiya et al., J. Med. Chem. 35: 4557-4561 (1992). Compound 11 of this invention was prepared as described above. CGS 21680 (Lot No. 89H4607) and adenosine (Lot No.123HO94) were purchased from Sigma. Krebs-Henseleit solution was prepared according to Standard Methods, and 0.9% saline was purchased from McGraw, Inc. (Lot NoJ8B246).

### Methods

Adult Sprague Dawley rats and Hartley guinea pigs of either sex weighing from 230 to 260 grams and 300 to 350 grams, respectively were used in this study. Animals were anesthetized by peritoneal injection of a cocktail containing ketamine and xylazine (ketamine 100 mg, xylazin 20 mg/ml). The chest was opened and the heart quickly removed. The heart was briefly rinse in ice-cold Krebs-Henseleit solution (see below), and the aorta cannulated. The heart was then perfused at a flow rate of 10 ml/min with modified Krebs-Henseleit (K-H) solution containing NaCl 117.9, KCI 4.5, CaCl₂ 2.5, MgSO₄ 1.18, KH₂PO₄ 1-18, pyruvate 2.0 mmo/L. The K-H solution (pH 7.4) was gassed continuously with 95% 0₂ and 5% C0₂ and warmed to 35±0.50 C. The heart was electrically paced at a fixed cycle length of 340 ms (250 beats/min) using a bipolar electrode place on the left atrium. The electrical stimuli were generated by a Grass stimulator (Model S48, W. Warwick, RI) and delivered through a Stimuli Isolation Unit (Model SIU5, Astro-Med, Inc., NY) as square-wave pulses of 3-msec in duration and amplitude of at least twice the threshold intensity.

Coronary perfusion pressure (CPP) was measured using a pressure transducer, connected to the aortic cannula via a T-connector positioned approximately 3 cm above the heart. Coronary perfusion pressure was monitored throughout the experiment and recorded either on a chart recorder (Gould Recorder 2200S) or a computerized recording system (PowerLab/4S, ADinstruments Pty Ltd, Australia). Only hearts with CPP ranging from 60 to 85 mmHg (in the absence of drugs) were used in the study. Coronary conductance (in ml/min/mmHg) was calculated as the ratio between coronary perfusion rate (10 ml/min) and coronary perfusion pressure.

In experiments in which A, adenosine receptor-mediated negative dromotropic effect was measured, atrial and ventricular surface electrograms were recorded during constant atrial pacing. The effect of various adenosine receptor agonists on atrioventricular conduction time was determined as described by Jenkins and Belardinelli *Circ.* Res. 63: 97-116 (1988).

Stock solutions of the compound of this invention (5mM) and CGS 21680 (5mM) were prepared in dimethyl sulfoxide (DMSO); purchased from Aldrich, PS 04253MS. A stock solution of adenosine (1 mg/ml) was prepared in saline. One concentration was made from the stock solution by dilution into saline to yield solution of either 2X10⁻⁴ or 2X10⁻⁵ M. These solutions were injected into the perfusion line of the apparatus as boluses of 20 µl. In some experiments the solutions were placed into a 30 ml glass syringe and the drugs were infused at rates necessary to achieve the desired perfusate concentrations (e.g, 10, 100 nM, etc).

### Coronary vasodilation of A_{2A} adenosine receptor agonists

Concentration-response relationships for the effect of Compound 11 of this invention (0.1 to 400 nM) and CGS21680 (0.1 to 10 nM) to increase coronary conductance were obtained. After control measurements of coronary perfusion pressure were recorded, progressive higher concentrations of the adenosine receptor agonists were administered until maximal coronary vasodilation was observed. The steady-state responses to each concentration of adenosine receptor agonists were recorded. In each heart of this series (4 to 6 hearts for each agonist) only one agonist and one concentration-response relationship was obtained.

To determine which adenosine receptor subtype (A₁ or A_{2A}) mediates the coronary vasodilation caused by compounds of this invention, the A₁ and A_{2A} adenosine receptor antagonists CPX and ZM241385, respectively, were used. Hearts (n=6) were exposed to the compound being tested (10 nM), and after the effect of this agonist reached steady-state, first CPX (60 nM), and then ZM241385 were added to the perfusate and the changes in CPP were recorded.

In isolated perfused hearts (n=36 rats and 18 guinea pigs) paced at constant atrial cycle length of 340 msec, adenosine, CGS21680, WRC0470, and Compound 11 of this invention caused a concentration-dependent increase in coronary conductance. CGS21680, WRC0470, and Compound 11 were all more potent agonists than adenosine.

### EXAMPLE 13

The present study was designed to test the hypothesis that there is an inverse relationship between the affinity (Kᵢ or pKᵢ) and duration of action of A_{2A} adenosine receptors (AdoR). Specifically, the aims of the study were to determine the relationship between the duration of the coronary vasodilation caused by a selected series of high and low affinity A_{2A}AdoR agonists in rat isolated hearts and anesthetized pigs; and the affinity of these agonists for A_{2A} AdoRs in pig striatum.

**Materials:** Rats (Sprague Dawley) were purchased from Simonen. Farm pigs were obtained from Division of Laboratory Animal Resources, University of Kentucky. Compounds 11, 12 and 14 of this invention were prepared as described in the methods above. YT-0146 was prepared as described in U.S. Patent No. 4,956,345, the specification of which is incorporated herein by reference. WRC-0470 was prepared as described in the literature (K. Niiya et al., J. Med. Chem. 35: 4557-4561 (1992). CGS21680 was purchased from Research Biochemicals, Inc. and Sigma and R-PIA (Lot No. WY-V-23) was purchased from Research Biochemicals, Inc. HENECA was a gift from Professor Gloria Cristalli of University of Camerino, Italy.

The anesthetic agents: Ketamine was purchased from Fort Dodge Animal Health. Xylazine was purchased from Bayer. Sodium pentobarbital was purchased from The Butler Co. Phenylephrine was purchased from Sigma. DMSO was purchased from Sigma and American Tissue Type Collections. Krebs-Henseleit solution was prepared according to standard methods, and 0.9% saline was purchased from McGraw, Inc.

### Rat isolated perfused heart preparation

Adult Sprague Dawley rats of either sex weighing from 230 to 260 grams were used in this study. Animals were anesthetized by peritoneal injection of a cocktail containing ketamine and xylazine (ketamine 100 mg, xylazine 20 mg/ml). The chest was opened and the heart quickly removed. The heart was briefly rinse in ice-cold Krebs-Henseleit solution (see below), and the aorta cannulated. The heart was then perfused at a flow rate of 10 ml/min with modified Krebs-Henseleit (K-H) solution containing NaCl 117.9, KCl 4.5, CaCl. 2.5, MgSO₄ 1.18, KH₂PO₄ 1.18, pyruvate 2.0 mmo/L. The K-H solution (pH 7.4) was gassed continuously with 95% O₂ and 5% CO₂ and warmed to 35±0.50°C. The heart was electrically paced at a fixed cycle length of 340 ms (250 beats/min) using a bipolar electrode place on the left atrium. The electrical stimuli were generated by a Grass stimulator (Model S48, W. Warwick, RI) and delivered through a Stimuli Isolation Unit (Model SIU5, Astro-Med, Inc., NY) as square-wave pulses of 3msec in duration and amplitude of at least twice the threshold intensity.

Coronary perfusion pressure (CPP) was measured using a pressure transducer, connected to the aortic cannula via a T-connector positioned approximately 3 cm above the heart. Coronary perfusion pressure was monitored throughout the experiment and recorded either on a chart recorder (Gould Recorder 2200S) or a computerized recording system (PowerLab/4S, ADInstruments Pty Ltd, Australia). Only hearts with CPP ranging from 60 to 85 mmHg (in the absence of drugs) were used in the study. Coronary conductance (in ml/min/mmHg) was calculated as the ratio between coronary perfusion rate (10 ml/min) and coronary perfusion pressure.

### Isolated perfused hearts

To determine the duration of the A_{2A}adenosine receptormediated coronary vasodilation caused by adenosine and adenosine receptor agonists, the agonists were administered interveneously by bolus injection. In each heart of this series (3 to 11 hearts for each agonist), boluses of adenosine (20 µl, 2x 10⁻⁴M), Compounds of this invention (20 to 40 µl, 2x 10⁻⁵ M), and other adenosine receptor agonists were injected into the perfusion line. The times to 50% *(t* 0.5) and 90% *(t* 0.9) reversal of the decrease in CPP were measured. Each heart was exposed to a maximum of three vasodilators.

**Relationship between affinity of various agonists for A**_{**2A**} **adenosine receptor and the reversal time of their effect to increase coronary conductance:** These experiments were performed to construct the relationship between the affinities of the various agonists for A_{2A}adenosine receptor and the duration of their respective effect on coronary conductance. Boluses of various agonists were injected into the perfusion line of rat isolated perfused hearts (n=4 to 6 for each agonist) and the time to 90% *(t* 0.9) reversal of the decrease in CPP measured. The affinities of the various agonists for A_{2A} adenosine receptor was determined in pig striatum membranes using a radioligand binding assay, as described above. The reversal time (t 0.9) of the decrease in CPP was plotted against their affinities (pKᵢ) for the

A_{2A} adenosine receptor.

### Results

Adenosine, the compounds of this invention and other adenosine derivatives were given as boluses into the perfusion line at concentrations that cause equal or near-equal increases in coronary conductance. Although adenosine and the agonists caused equal maximal increases in coronary conductance the duration of their effect was markedly different. The duration of the effect of adenosine. The duration effect of Compounds 11, 12 ans 14 fell within the middle of the group of compounds tested whereas that of CGS21680 and WRC0470 were the longest. The durations of the coronary vasodilation caused by adenosine, the compounds of this invention and other agonists measured as the time to 50% and 90% *(t* 0.5 and t 0.9, respectively) reversal of the increases in coronary conductance are summarized in Table 7, below

**Table 7 -**

| **Reversal Time Of Coronary Vasodilation by Adenosine and adenosine receptor agonists in Rat Isolated Perfused Hearts** | | | |
|---|---|---|---|
| Agonist | ***t* 0.5 (min)** | ***t* 0.9 (min)** | **n** |
| Adenosine | 1.06±0.1 | 5.6±0.8 | 11 |
| HENECA | 28.6±1.1 | 32.8±3.1 | 3 |
| R-PIA | 7.9±0.1 | 12.6±0.8 | 3 |
| CGS21680 | 14.5±0.9 | 19.5±0.9 | 3 |
| YT-146 | 17.7±1.0 | 28.5±4.0 | 3 |
| Compound 11 | 17.5±1.2 | 23.2±2.1 | 4 |
| Compound 14 | 16.1±0.1 | 21.8±2.0 | 3 |
| WRC-0470 | 21.9±0.9 | 27.9±1.4 | 6 |
| Compound 12 | 16.7±0.5 | 25.6±2.3 | 3 |
| Time (in minutes) to 50% and 90% (t 0.5 and t 0.9, respectively) reversal of the increases in coronary conductance caused by adenosine and adenosine receptor agonists. Values are the means±SEM of single determinations in each of the preparations (n). | | | |

The reversal time of coronary vasodilation was dependent on the affinity of the adenosine derivatives for brain striatum A_{2A} receptors. There was a significant (P < 0.05) inverse relationship ( r = 0.87) between the affinity (PKᵢ) of the agonists for the A_{2A}AdoR and the reversal time (t 0.9) of the coronary vasodilation caused by the same agonists.

## Claims

1. A compound having the formula: 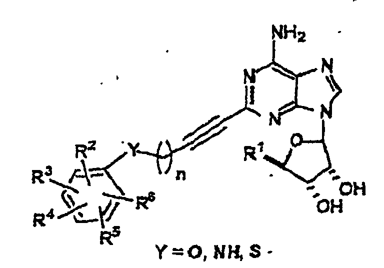 wherein n is 1 to 2;
Y is O, NH, or S;
R¹ is CH₂OH, or C(=O)NR⁷R²;
R², R³, R⁴, R⁵, and R⁶ are each individually selected from the group consisting of hydrogen, halo, NO₂, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂ NR²⁰COR²², NR²⁰CO₂R²², NR²⁰CON(R²⁰)₂, COR²⁰, CO₂R²⁰, CON(R²⁰)₂, CONR²⁰SO₂R²⁰, NR²⁰SO₂R²², SO₂NR²⁰CO₂R²², OCONR²⁰SO₂R²², OC(O)R²⁰, C(O)OCH₂OC(O)R²⁰, OCON(R²⁰)₂, C₁₋₁₅ alkyl, C₂₋₁₅ alkenyl, C₂₋₁₅ alkynyl, C₁₋₁₅ alkoxy, heterocyclyl, aryl, and heteroaryl, wherein the alkyl, alkenyl, alkynyl, alkoxy, aryl, heterocyclyl, and heteroaryl are optionally substituted with from 1 to 3 substituents independently selected from the group consisting of halo, NO₂, heterocyclyl, aryl, heteroaryl, CF₃, CN, OR²⁰, SR²⁰, N(R²⁰)₂, S(O)R²², SO₂R²², SO₂N(R²⁰)₂, SO₂NR²⁰COR²²; SO₂NR²⁰CO₂R²², SO₂NR²⁰CON(R²⁰)₂, N(R²⁰)₂ NR²⁰COR²², NR²⁰CO₂R²², NR²⁰CON(R²⁰)₂, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰, and OCON(R²⁰)₂ with each optional heteroaryl, aryl, and heterocyclyl substituent further optionally substituted with halo, NO₂, alkyl, CF₃, amino, mono- or di-alkylamino, alkyl, aryl, heteroaryl amide, NCOR²², NR²⁰SO₂R²², COR²⁰, CO₂R²⁰, CON(R²⁰)₂, NR²⁰CON(R²⁰)₂, OC(O)R²⁰, OC(O)N(R²⁰)₂, SR²⁰, S(O)R²², SO₂R²², SO₂N(R²⁰)₂, CN, and OR²⁰;
R⁷ and R⁸ are each individually selected from H, and C₁₋₁₅ alkyl optionally substituted with from 1 to 2 substituents, each substituent individually selected from the group consisting of halo, NO₂, heterocyclyl, aryl, heteroaryl, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂ NR²⁰COR²², NR²⁰CO₂R²², NR²⁰CON(R²⁰)₂, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰, and OCON(R²⁰)₂ with each optional heteroaryl, aryl, and heterocyclyl substituent further optionally substituted with halo, NO₂, alkyl, CF₃, amino, mono- or di- alkylamino, alkyl or aryl or heteroaryl amide, NCOR²², NR²⁰SO₂R²², COR²⁰, CO₂R²⁰, CON(R²⁰)₂, NR²⁰CON(R²⁰)₂, OC(O)R²⁰, OC(O)N(R³⁰)₂, SR²⁰, S(O)R²², SO₂R²², SO₂N(R²⁰)₂, CN, and OR²⁰;
R²⁰ is selected from the group consisting of H, C₁₋₁₅ alkyl, C₂₋₁₅ alkenyl, C₂₋₁₅ alkynyl, heterocyclyl, aryl, and heteroaryl, wherein the alkyl; alkenyl, alkynyl, heterocyclyl, aryl, and heteroaryl are each optionally substituted with from 1 to 3 substituents individually selected from halo, alkyl, mono- or dialkylamino, alkyl or aryl or heteroaryl amide, CN, -O-C₁₋₆ alkyl, CF₃, aryl, and heteroaryl; and
R²² is selected from the group consisting of C₁₋₁₅ alkyl, C₂₋₁₅ alkenyl, C₂₋₁₅ alkynyl, heterocyclyl, aryl, and heteroaryl, which alkyl, alkenyl, alkynyl, heterocyclyl, aryl, and heteroaryl are each optionally substituted with 1 to 3 substituents independently selected from halo, alkyl, mono- or dialkylamino, alkyl or aryl or heteroaryl amide, CN, O-C₁₋₆ alkyl, CF₃, and heteroaryl.

2. The compound of claim 1 wherein R², R³, R⁴, R⁵, and R⁶ are each individually selected from the group consisting of hydrogen, halo, NO₂, CF₃, CN, OR²⁰, SR²⁰, N(R²⁰)₂, S(O)R²², SO₂R²², SO₂N(R²⁰)₂, NR²⁰CO₂R²², NR²⁰CON(R²⁰)₂, COR²⁰, CO₂R²⁰, CON(R²⁰)₂, NR²⁰SO₂R²², OC(O)R²⁰, OCON(R²⁰)₂, C₁₋₁₅ alkyl, C₂₋₁₅ alkenyl, C₂₋₁₅ alkynyl, heterocyclyl, aryl, and heteroaryl, wherein the alkyl, alkenyl, alkynyl, aryl, heterocyclyl, and heteroaryl are each optionally substituted with from 1 to 3 substituents independently selected from the group consisting of halo, NO₂, heterocyclyl, aryl, heteroaryl, CF₃, CN, OR²⁰, SR²⁰, N(R²⁰)₂, S(O)R²², SO₂R²², SO₂N(R²⁰)₂, NR²⁰CO₂R²², NR²⁰CON(R²⁰)₂, COR²⁰, CO₂R²⁰, CON(R²⁰)₂, NR²⁰SO₂R²², OC(O)R²⁰, and OCON(R²⁰)₂ with each optional heteroaryl, aryl, and heterocyclyl substituent further optionally substituted with halo, alkyl, CF₃, mono- or di- alkylamino, SR²⁰, CN, and OR²⁰ ;
R⁷ and R⁸ are each individually selected from H, and C₁₋₁₅ alkyl optionally substituted with from 1 to 2 substituents independently selected from the group consisting of aryl, heteroaryl, CF₃, and OR²⁰, wherein each optional heteroaryl, or aryl substituent is optionally substituted with halo, alkyl, and CF₃; and
R²⁰ is selected from the group consisting of H, C₁₋₆ alkyl, C₂₋₆ alkenyl, C₂₋₆ alkynyl, and aryl wherein each alkyl, alkeayl, alkynyl, and aryl are further optionally substituted with from 1 to 3 substituents independently selected from halo, alkyl, CN, O-C₁₋₆ alkyl, and CF₃.

3. The compound of claim 1 wherein R², R³, R⁴, R⁵, and R⁶ are individually selected from the group consisting of hydrogen, halo, NO₂, CF₃, CN, OR²⁰, SR²⁰, N(R²⁰)₂, C₁₋₁₅ alkyl, C₂₋₁₅ alkenyl, heterocyclyl, aryl, and heteroaryl which alkyl, alkenyl, aryl, heterocyclyl, and heteroaryl are optionally substituted with from 1 to 3 substituents independently selected from the group consisting of halo, NO₂, aryl, CF₃, CN, OR²⁰, SR²⁰, N(R²⁰)₂, with each optional, aryl, further optionally substituted with halo, alkyl, CF₃, SR²⁰, CN, and OR²⁰ ;
R⁷ and R³ are each independently selected from H, and C₁₋₁₅ alkyl substituted with from I to 2 CF₃ groups, and
R²⁰ is selected from the group consisting of H, and C₁₋₄ alkyl, wherein the C₁₋₄ alkyl is optionally substituted with from 1 to 3 substituents independently selected from halo, alkyl, CN, O-C₁₋₃ alkyl, and CF₃.

4. The compound of claim 1 wherein R², R³, R⁴, R⁵, and R⁶ are each individually selected from the group consisting of hydrogen, halo, CF₃, CN, OR²⁰, SR¹⁰, N(R²⁰)₂, C₁₋₁₅ alkyl, heterocyclyl, and aryl, wherein the alkyl, aryl, and heterocyclyl are optionally substituted with 1 to 2 substituents independently selected-from the group consisting of halo, aryl, CF₃, CN, OR²⁰, SR²⁰, N(R²⁰)₂ and wherein each optional aryl substituent is further optionally substituted with halo, alkyl, CF₃, CN, and OR²⁰;
R⁷ and R⁸ each individually selected from H, and C₁₋₁₅ alkyl, and
R²⁰ is selected from the group consisting of H, and C₁₋₃ alkyl, wherein the C₁₋₃ alkyl is optionally substituted with from 1 to 3 substituents independently selected from halo, alkyl, CN, and CF₃.

5. The compound of claim 1 wherein R², R³, R⁴, R⁵, and R⁶ are each individually selected from the group consisting of hydrogen, halo, CF₃, CN, OR²⁰, SR²⁰, and N(R²⁰)₂, C₁₋₁₅ alkyl, heterocyclyl, and aryl, wherein the alkyl, aryl, and heterocyclyl are optionally substituted with from 1 to 2 substituents independently selected from the group consisting of halo; aryl, CF₃, CN, OR²⁰, SR²⁰, and N(R²⁰)₂, and wherein each optional aryl substituent is further optionally substituted with halo, alkyl, CN, and CF₃;
R⁷ and R⁸ are each individually selected from H, and C₁₋₈ alkyl; and
R²⁰ is selected from the group consisting of H, and C₁₋₃ alkyl.

6. The compound of claim 5 wherein at least one substituent selected from the group consisting of R², R³, R⁴, R⁵, and R⁶ is hydrogen.

7. The compound of claim 5 wherein at least two substituents selected from the group consisting of R², R³, R⁴, R⁵, and R⁶ are hydrogen.

8. The compound of claim 5 wherein at least three substituents selected from the group consisting of R², R³, R⁴, R⁵, and R⁶ are hydrogen, wherein Y is O and wherein n is 1.

9. The compound of claim 8 wherein R¹ is CH₂OH;
R², R³, R⁴, R⁵, and R⁶ are each individually selected from the group consisting of hydrogen, halo, CF₃, CN, OR²⁰, C₁₋₈ alkyl, heterocyclyl, and aryl, wherein the alkyl, aryl, and heterocyclyl are optionally substituted with one substituent selected from the group consisting of halo, aryl, CF₃, CN, and OR²⁰, and wherein each optional aryl substituent is further optionally substituted with halo, alkyl, CN, and CF₃; and
R²⁰ is selected from the group consisting of H, and C₁₋₃ alkyl.

10. The compound of claim 8 wherein R¹ is CH₂OH;
R², R³, R⁴, R⁵, and R⁶ are each individually selected from the group consisting of hydrogen, halo, CF₃, CN, OR²⁰, C₁₋₆ alkyl, heterocyclyl, and aryl, wherein the alkyl, and aryl are optionally substituted with one substituent selected from the group consisting of halo, aryl, CF₃, CN, and OR²⁰; and
R³⁰ is selected from the group consisting of H, and C₁₋₃ alkyl.

11. The compound of claim 8 wherein R¹ is CH₂OH;
R¹, R³, R⁴, R⁵, and R⁶ are each individually selected from the group consisting of hydrogen, halo, CF₃, CN, OR²⁰, C₁₋₄ alkyl, heterocyclyl, and aryl, wherein the alkyl and aryl are optionally substituted with aryl; and
R²⁰ is selected from the group consisting of H and C₁₋₃ alkyl.

12. The compound of claim 8 wherein R¹ is CH₂OH;
R², R³, R⁴, R⁵, and R⁶ are each individually selected from the group consisting of hydrogen, halo, CF₃, CN, OR²⁰, C₁₋₄ alkyl optionally substituted with aryl and heterocyclyl wherein the heterocyclyl is a fused five to seven membered ring containing 1 to 3 heteroatoms selected from O, N, S, and combinations thereof; and
R²⁰ is selected from the group consisting of H, and C₁₋₃ alkyl.

13. The compound of claim 8 wherein R¹ is CH₂OH;
R², R³, R⁴, R⁵, and R⁶ are each individually selected from the group consisting of hydrogen, halo, CF₃, CN, OR²⁰, C₁₋₄ alkyl and aryl optionally substituted with aryl and heterocyclyl wherein the heterocyclyl is a fused five membered ring containing two oxygen atoms; and
R²⁰ is selected from the group consisting of H, and C₁₋₃ alkyl.

14. The compound of claim 8 wherein R¹ is CH₂OH, and R², R³, R⁴, R⁵, and R⁶ are each individually selected from the group consisting of hydrogen, CN, and C₁₋₄ alkyl optionally substituted with aryl and a fused five membered ring containing two oxygen atoms with points of attachments at the 2 and 3 position.

15. The compound of claim 14 wherein four substituents selected from the group consisting of R², R³, R⁴, R⁵, and R⁶ are hydrogen.

16. The compound of claim 8 wherein R¹ is C(O)NHEt;
R², R³, R⁴, R⁵, and R⁶ are each individually selected from the group consisting of hydrogen, halo, CF₃, CN, OR²⁰, C₁₋₈ alkyl, heterocyclyl, and aryl wherein the alkyl, aryl, and heterocyclyl are optionally substituted with a substituent independently selected from the group consisting of halo, aryl, CF₃, CN, and OR²⁰, wherein each optional aryl substituent is further optionally substituted with halo, alkyl, CN, and CF₃; and
R²⁰ is selected from the group consisting of H, and C₁₋₃ alkyl

17. The compound of claim 8 wherein R¹ is C(O)NHEt;
R², R³, R⁴, R⁵, and R⁶ are each individually selected from the group consisting of hydrogen, halo, CF₃, CN, OR²⁰, C₁₋₆ alkyl, heterocyclyl, and aryl, wherein the alkyl and aryl are optionally substituted with one substituent selected from the group consisting of halo, aryl, CF₃, CN, and OR²⁰; and
R²⁰ is selected from the group consisting of H, and C₁₋₃ alkyl.

18. The compound of claim 8 wherein R¹ is C(O)NHEt;
R², R³, R⁴, R⁵, and R⁶ are each individually selected from the group consisting of hydrogen, halo, CF₃, CN, OR²⁰, C₁₋₄ alkyl, heterocyclyl, and aryl, wherein the alkyl and aryl are optionally substituted with one aryl substituent; and
R²⁰ is selected from the group consisting of H, and C₁₋₃ alkyl.

19. The compound of claim 8 wherein R¹ is C(O)NHEt; R², R³, R⁴, R⁵, and R⁶ are each individually selected, from the group consisting of hydrogen, halo, CF₃, CN, OR²⁰, aryl, C₁₋₄ alkyl, optionally substituted with one substituent selected from aryl, and a fused five to seven membered ring containing 1-3 heteroatoms selected from O, N, S; and
R²⁰ is selected from the group consisting of H, and C₁₋₃ alkyl.

20. The compound of claim 8 wherein R¹ is C(O)NHEt;
R², R³, R⁴, R⁵, and R⁶ are each individually selected from the group consisting of hydrogen, halo, CF₃, CN, OR²⁰, aryl, and C₁₋₄ alkyl that is optionally substituted with one substituent selected from the group consisting of aryl and a fused five membered ring containing two oxygen atoms; and
R²⁰ is selected from the group consisting of H, and C₁₋₃ alkyl.

21. The compound of claim 8 wherein R¹ is C(O)NHEt;
R², R³, R⁴, R⁵, and R⁶ are each individually selected from the group consisting of hydrogen, CN, aryl, and C₁₋₄ alkyl that is optionally substituted with one substituent selected from aryl, and a fused five membered ring containing two oxygen atoms with points of attachment at the 2 and 3 position.

22. The compound of claim 21 wherein four substitutents selected from the group consisting of R², R³, R⁴, R⁵, and R⁶ are hydrogen.

23. The compound matter of claim 1 wherein the compound is selected from the group consisting of (4S,2R,3R,5R)-2-{6-amino-2-[3-(2-phenylphenoxy)prop-1-ynyl]purin-9-yl}-5-(hydroxymethyl)oxolane-3,4-diol, (4S,2R,3R,5R)-2-[6-amino-2-(3-phenoxyprop-1-ynyl)purin-9-yl]-5-(hydroxymethyl)oxolane-3,4-diol, 4-(3-{9-[(4S,2R,3R,5R)-3,4-dihydroxy-5-(hydroxymethyl)oxolan-2-yl]-6-aminopurin-2-yl} prop-2-ynyloxy)benzenecarbonitrile, (4S,2R,3R,5R)-2-{6-amino-2-[3-(4-phenylphenoxy)prop-1-ynyl]purin-9-yl}-5-(hydroxymethyl)oxolane-3,4-diol, (4S,2R,3R,5R)-2-(6-amino-2-{3-[2-benzylphenoxy]prop-1-ynyl} purin-9-yl)-5-(hydroxymethyl)oxolane-3,4-diol, 2-[2-(3-(2H-benzo[2,3-d]1,3-dioxolen-4-yloxy)prop-1-ynyl)-6-aminopurin-9-yl](4S,2R,3R,5R)-5-(hydroxymethyl)oxolane-3,4-diol, (4S,2R,3R,5R)-2-(6-amino-2-{3-[3,5-bis(tert-butyl)phenoxy]prop-1-ynyl} purin-9-yl)-5-(hydroxymethyl)oxolane-3,4-diol, and mixtures thereof.

24. The use of a compound of claim 1, for the preparation of a pharmaceutical compostion for stimulating coronary vasodilatation in a mammal .

25. The use of claim 24 wherein the pharmaceutical composition is for administering a therapeutically effective amount of the compound of claim 1 ranging from about 0.01 to about 100 mg/kg weight of the mammal.

26. The use of claim 24 wherein the mammal is a human.

27. A pharmaceutical composition comprising the compound of claim 1 and one or more pharmaceutical excipients.

28. The pharmaceutical composition of claim 27 wherein the pharmaceutical composition is in the form of a solution.

29. The pharmaceutical composition of claim 27 wherein the composition is useful as an anti-inflammatory, in adjunctive therapy with angioplasty, as a platelet aggregation inhibitor, and as an inhibitor of platelet and neutrophil activation.

30. The pharmaceutical composition of claim 27 including a pro-drug of a compound of claim 1.

## Patentansprüche

1. Verbindung der Formel 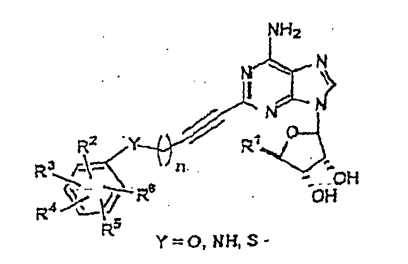 worin n 1 bis 2 ist,
Y O, NH oder S ist,
R¹ CH₂OH oder C(=O)NR⁷R⁸ ist,
R², R³, R⁴, R⁵ und R⁶ jeweils unabhängig ausgewählt sind aus der Gruppe bestehend aus Wasserstoff-, Halogenatom, NO₂, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂, NR²⁰COR²², NR²⁰CO₂R²², NR²⁰CON(R²⁰)₂, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰, OCON(R²⁰)₂, C₁₋₁₅-Alkyl, C₂₋₁₅-Alkenyl, C₂₋₁₅-Alkinyl, C₁₋₁₅-Alkoxy, Heterocyclyl, Aryl und Heteroaryl, worin die Alkyl-, Alkenyl-, Alkinyl-, Alkoxy-, Aryl-, Heterocyclyl- und Heteroarylgruppen gegebenenfalls mit 1 bis 3 Substituenten substituiert sind, die unabhängig ausgewählt sind aus der Gruppe bestehend aus Halogenatom, NO₂, Heterocyclyl, Aryl, Heteroaryl, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂, NR²⁰COR²², NR²⁰CO₂R²², NR²⁰CON(R²⁰)₂, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰ und OCON(R²⁰)₂, wobei jeder optionale Heteroaryl-, Aryl- und Heterocyclylsubstituent ferner gegebenenfalls mit Halogenatom, NO₂, Alkyl, CF₃, Amino, Monooder Dialkylamino, Alkyl-, Aryl-, Heteroarylamid, NCOR²², NR²⁰SO₂R²², COR²⁰, CO₂R²⁰, CON(R²⁰)₂, NR²⁰CON(R²⁰)₂, OC(O)R²⁰, OC(O)N(R²⁰)₂, SR²⁰, S(O)R²², SO₂R²², SO₂N(R²⁰)₂, CN und OR²⁰ substituiert ist,
R⁷ und R⁸ jeweils unabhängig ausgewählt sind aus H und C₁₋₁₅-Alkyl, gegebenenfalls substituiert mit 1 bis 2 Substituenten, wobei jeder Substituent unabhängig ausgewählt ist aus der Gruppe bestehend aus Halogenatom, NO₂, Heterocyclyl, Aryl, Heteroaryl, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂NR²⁰CON(R²⁰)₂, N(R²⁰)₂, NR²⁰COR²², NR²⁰CO₂R²², NR²⁰CON(R²⁰)₂, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰ und OCON(R²⁰)₂, wobei jeder optionale Heteroaryl-, Aryl- und Heterocyclylsubstituent ferner gegebenenfalls mit Halogenatom, NO₂, Alkyl, CF₃, Amino, Monooder Dialkylamino, Alkyl- oder Aryl- oder Heteroarylamid, NCOR²², NR²⁰SO₂R²², COR²⁰, CO₂R²⁰, CON(R²⁰)₂, NR²⁰CON(R²⁰)₂, OC(O)R²⁰, OC(O)N(R²⁰)₂, SR²⁰, S(O)R²², SO₂R²², SO₂N(R²⁰)₂, CN und OR²⁰ substituiert ist,
R²⁰ ausgewählt ist aus der Gruppe bestehend aus H, C₁₋₁₅-Alkyl, C₂₋₁₅-Alkenyl, C₂₋₁₅-Alkinyl, Heterocyclyl, Aryl und Heteroaryl, worin die Alkyl-, Alkenyl-, Alkinyl-, Heterocyclyl-, Aryl- und Heteroarylgruppen jeweils gegebenenfalls mit 1 bis 3 Substituenten substituiert sind, die unabhängig ausgewählt sind aus Halogenatom, Alkyl, Mono- oder Dialkylamino, Alkyl- oder Aryl- oder Heteroarylamid, CN, -O-C₁₋₆-Alkyl, CF₃, Aryl und Heteroaryl, und
R²² ausgewählt ist aus der Gruppe bestehend aus C₁₋₁₅-Alkyl, C₂₋₁₅-Alkenyl, C₂₋₁₅-Alkinyl, Heterocyclyl, Aryl und Heteroaryl, wobei die Alkyl-, Alkenyl-, Alkinyl-, Heterocyclyl-, Aryl- und Heteroarylgruppen jeweils gegebenenfalls mit 1 bis 3 Substituenten substituiert sind, die unabhängig aus Halogenatom, Alkyl, Mono- oder Dialkylamino, Alkyl- oder Aryl- oder Heteroarylamid, CN, O-C₁₋₆-Alkyl, CF₃ und Heteroaryl ausgewählt sind.

2. Verbindung nach Anspruch 1, worin R², R³, R⁴, R⁵ und R⁶ jeweils unabhängig ausgewählt sind aus der Gruppe bestehend aus Wasserstoff-, Halogenatom, NO₂, CF₃, CN, OR²⁰, SR²⁰, N(R²⁰)₂, S(O)R²², SO₂R²², SO₂N(R²⁰)₂, NR²⁰CO₂R²², NR²⁰CON(R²⁰)₂, COR²⁰, CO₂R²⁰, CON(R²⁰)₂, NR²⁰SO₂R²², OC(O)R²⁰, OCON(R²⁰)₂, C₁₋₁₅-Alkyl, C₂₋₁₅-Alkenyl, C₂₋₁₅-Alkinyl, Heterocyclyl, Aryl und Heteroaryl, worin die Alkyl-, Alkenyl-, Alkinyl-, Aryl-, Heterocyclyl- und Heteroarylgruppen jeweils gegebenenfalls mit 1 bis 3 Substituenten substituiert sind, die unabhängig ausgewählt sind aus der Gruppe bestehend aus Halogenatom, NO₂, Heterocyclyl, Aryl, Heteroaryl, CF₃, CN, OR²⁰, SR²⁰, N(R²⁰)₂, S(O)R²², SO₂R²², SO₂N(R²⁰)₂, NR²⁰CO₂R²², NR²⁰CON(R²⁰)₂, COR²⁰, CO₂R²⁰, CON(R²⁰)₂, NR²⁰SO₂R²², OC(O)R²⁰ und OCON(R²⁰)₂, wobei jeder optionale Heteroaryl-, Aryl- und Heterocyclylsubstituent ferner gegebenenfalls mit Halogenatom, Alkyl, CF₃, Mono- oder Dialkylamino, SR²⁰, CN und OR²⁰ substituiert ist,
R⁷ und R⁸ jeweils unabhängig aus H und C₁₋₁₅-Alkyl ausgewählt sind, gegebenenfalls substituiert mit 1 bis 2 Substituenten, die unabhängig ausgewählt sind aus der Gruppe bestehend aus Aryl, Heteroaryl, CF₃ und OR²⁰, worin jeder optionale Heteroaryl- oder Arylsubstituent gegebenenfalls mit Halogenatom, Alkyl und CF₃ substituiert ist, und
R²⁰ ausgewählt ist aus der Gruppe bestehend aus H, C₁₋₆-Alkyl, C₂₋₆-Alkenyl, C₂₋₆-Alkinyl und Aryl, worin jede Alkyl-, Alkenyl-, Alkinyl- und Arylgruppe ferner gegebenenfalls mit 1 bis 3 Substituenten substituiert ist, die unabhängig aus Halogenatom, Alkyl, CN, O-C₁₋₆-Alkyl und CF₃ ausgewählt sind.

3. Verbindung nach Anspruch 1, worin R², R³, R⁴, R⁵ und R⁶ unabhängig ausgewählt sind aus der Gruppe bestehend aus Wasserstoff-, Halogenatom, NO₂, CF₃, CN, OR²⁰, SR²⁰, N(R²⁰)₂, C₁₋₁₅-Alkyl, C₂₋₁₅-Alkenyl, Heterocyclyl, Aryl und Heteroaryl, wobei die Alkyl-, Alkenyl-, Aryl-, Heterocyclyl- und Heteroarylgruppen gegebenenfalls mit 1 bis 3 Substituenten substituiert sind, die unabhängig ausgewählt sind aus der Gruppe bestehend aus Halogenatom, NO₂, Aryl, CF₃, CN, OR²⁰, SR²⁰, N(R²⁰)₂, wobei jeder optionale Arylsubstituent gegebenenfalls ferner mit Halogenatom, Alkyl, CF₃, SR²⁰, CN und OR²⁰ substituiert ist,
R⁷ und R⁸ jeweils unabhängig ausgewählt sind aus H und C₁₋₁₅-Alkyl, substituiert mit 1 bis 2 CF₃-Gruppen, und
R²⁰ ausgewählt ist aus der Gruppe bestehend aus H und C₁₋₄-Alkyl, worin die C₁₋₄-Alkylgruppe gegebenenfalls mit 1 bis 3 Substituenten substituiert ist, die unabhängig aus Halogenatom, Alkyl, CN, O-C₁₋₃-Alkyl und CF₃ ausgewählt sind.

4. Verbindung nach Anspruch 1, worin R², R³, R⁴, R⁵ und R⁶ jeweils unabhängig ausgewählt ist aus der Gruppe bestehend aus Wasserstoff-, Halogenatom, CF₃, CN, OR²⁰, SR²⁰, N(R²⁰)₂, C₁₋₁₅-Alkyl, Heterocyclyl und Aryl, worin die Alkyl-, Aryl- und Heterocyclylgruppen gegebenenfalls mit 1 bis 2 Substituenten substituiert sind, die unabhängig ausgewählt sind aus der Gruppe bestehend aus Halogenatom, Aryl, CF₃, CN, OR²⁰, SR²⁰, N(R²⁰)₂, und worin jeder optionale Arylsubstituent ferner gegebenenfalls mit Halogenatom, Alkyl, CF₃, CN und OR²⁰ substituiert ist,
R⁷ und R⁸ jeweils unabhängig ausgewählt sind aus H und C₁₋₁₅-Alkyl und
R²⁰ ausgewählt ist aus der Gruppe bestehend aus H und C₁₋₃-Alkyl, worin die C₁₋₃-Alkylgruppe gegebenenfalls mit 1 bis 3 Substituenten substituiert ist, die unabhängig aus Halogenatom, Alkyl, CN und CF₃ ausgewählt sind.

5. Verbindung nach Anspruch 1, worin R², R³, R⁴, R⁵ und R⁶ jeweils unabhängig ausgewählt sind aus der Gruppe bestehend aus Wasserstoff-, Halogenatom, CF₃, CN, OR²⁰, SR²⁰ und N(R²⁰)₂, C₁₋₁₅-Alkyl, Heterocyclyl und Aryl, worin die Alkyl-, Aryl- und Heterocyclylgruppen gegebenenfalls mit 1 bis 2 Substituenten substituiert sind, die unabhängig ausgewählt sind aus der Gruppe bestehend aus Halogenatom, Aryl, CF₃, CN, OR²⁰, SR²⁰ und N(R²⁰)₂, und worin jeder optionale Arylsubstituent ferner gegebenenfalls mit Halogenatom, Alkyl, CN und CF₃ substituiert ist,
R⁷ und R⁸ jeweils unabhängig ausgewählt sind aus H und C₁₋₈-Alkyl und
R²⁰ ausgewählt ist aus der Gruppe bestehend aus H und C₁₋₃-Alkyl.

6. Verbindung nach Anspruch 5, worin mindestens ein Substituent aus der Gruppe bestehend aus R², R³, R⁴, R⁵ und R⁶ ein Wasserstoffatom ist.

7. Verbindung nach Anspruch 5, worin mindestens zwei Substituenten aus der Gruppe bestehend aus R², R³, R⁴, R⁵ und R⁶ Wasserstoffatome sind.

8. Verbindung nach Anspruch 5, worin mindestens drei Substituenten aus der Gruppe bestehend aus R², R³, R⁴, R⁵ und R⁶ Wasserstoffatome sind, Y ein Sauerstoffatom und n 1 ist.

9. Verbindung nach Anspruch 8, worin R¹ CH₂OH ist, R², R³, R⁴, R⁵ und R⁶ jeweils unabhängig ausgewählt sind aus der Gruppe bestehend aus Wasserstoff-, Halogenatom, CF₃, CN, OR²⁰, C₁₋₈-Alkyl, Heterocyclyl und Aryl, worin die Alkyl-, Aryl- und Heterocyclylgruppen gegebenenfalls mit einem Substituent substituiert sind, der ausgewählt ist aus der Gruppe bestehend aus Halogenatom, Aryl, CF₃, CN und OR²⁰, und worin jeder optionale Arylsubstituent ferner gegebenenfalls mit Halogenatom, Alkyl, CN und CF₃ substituiert ist und
R²⁰ ausgewählt ist aus der Gruppe bestehend aus H und C₁₋₃-Alkyl.

10. Verbindung nach Anspruch 8, worin R¹ CH₂OH ist,
R², R³, R⁴, R⁵ und R⁶ jeweils unabhängig ausgewählt sind aus der Gruppe bestehend aus Wasserstoff-, Halogenatom, CF₃, CN, OR²⁰, C₁₋₆-Alkyl, Heterocyclyl und Aryl, worin die Alkylund Arylgruppen gegebenenfalls mit einem Substituent substituiert sind, der ausgewählt ist aus der Gruppe bestehend aus Halogenatom, Aryl, CF₃, CN und OR²⁰, und
R²⁰ ausgewählt ist aus der Gruppe bestehend aus H und C₁₋₃-Alkyl.

11. Verbindung nach Anspruch 8, worin R¹ CH₂OH ist,
R², R³, R⁴, R⁵ und R⁶ jeweils unabhängig ausgewählt sind aus der Gruppe bestehend aus Wasserstoff-, Halogenatom, CF₃, CN, OR²⁰, C₁₋₄-Alkyl, Heterocyclyl und Aryl, worin die Alkylund Arylgruppen gegebenenfalls mit Aryl substituiert sind, und
R²⁰ ausgewählt ist aus der Gruppe bestehend aus H und C₁₋₃-Alkyl.

12. Verbindung nach Anspruch 8, worin R¹ CH₂OH ist,
R², R³, R⁴, R⁵ und R⁶ jeweils unabhängig ausgewählt sind aus der Gruppe bestehend aus Wasserstoff-, Halogenatom, CF₃, CN, OR²⁰, C₁₋₄-Alkyl, gegebenenfalls substituiert mit Aryl und Heterocyclyl, worin die Heterocyclylgruppe ein fusionierter 5- bis 7-gliedriger Ring mit 1 bis 3 Heteroatomen, ausgewählt aus O, N, S und Kombinationen davon, ist, und
R²⁰ ausgewählt ist aus der Gruppe bestehend aus H und C₁₋₃-Alkyl.

13. Verbindung nach Anspruch 8, worin R¹ CH₂OH ist,
R², R³, R⁴, R⁵ und R⁶ jeweils unabhängig ausgewählt sind aus der Gruppe bestehend aus Wasserstoff-, Halogenatom, CF₃, CN, OR²⁰, C₁₋₄-Alkyl und Aryl, gegebenenfalls substituiert mit Aryl und Heterocyclyl, worin die Heterocyclylgruppe ein fusionierter 5-gliedriger Ring mit zwei Sauerstoffatomen ist, und
R²⁰ ausgewählt ist aus der Gruppe bestehend aus H und C₁₋₃-Alkyl.

14. Verbindung nach Anspruch 8, worin R¹ CH₂OH ist und R², R³, R⁴, R⁵ und R⁶ jeweils unabhängig ausgewählt sind aus der Gruppe bestehend aus Wasserstoffatom, CN und C₁₋₄-Alkyl, gegebenenfalls substituiert mit Aryl und einem fusionierten 5-gliedrigen Ring mit zwei Sauerstoffatomen, wobei sich die Verknüpfungsstellen an der 2- und 3-Position befinden.

15. Verbindung nach Anspruch 14, worin vier Substituenten aus der Gruppe bestehend aus R², R³, R⁴, R⁵ und R⁶ Wasserstoffatome sind.

16. Verbindung nach Anspruch 8, worin R¹ C(O)NHEt ist,
R², R³, R⁴, R⁵ und R⁶ jeweils unabhängig ausgewählt sind aus der Gruppe bestehend aus Wasserstoff-, Halogenatom, CF₃, CN, OR²⁰, C₁₋₈-Alkyl, Heterocyclyl und Aryl, worin die Alkyl-, Aryl- und Heterocyclylgruppen gegebenenfalls mit einem Substituent substituiert sind, der unabhängig ausgewählt ist aus der Gruppe bestehend aus Halogenatom, Aryl, CF₃, CN und OR²⁰, worin jeder optionale Arylsubstituent ferner gegebenenfalls mit Halogenatom, Alkyl, CN und CF₃ substituiert ist und
R²⁰ ausgewählt ist aus der Gruppe bestehend aus H und C₁₋₃-Alkyl.

17. Verbindung nach Anspruch 8, worin R¹ C(O)NHEt ist,
R², R³, R⁴, R⁵ und R⁶ jeweils unabhängig ausgewählt sind aus der Gruppe bestehend aus Wasserstoff-, Halogenatom, CF₃, CN, OR²⁰, C₁₋₆-Alkyl, Heterocyclyl und Aryl, worin die Alkylund Arylgruppen gegebenenfalls mit einem Substituent substituiert sind, der ausgewählt ist aus der Gruppe bestehend aus Halogenatom, Aryl, CF₃, CN und OR²⁰, und
R²⁰ ausgewählt ist aus der Gruppe bestehend aus H und C₁₋₃-Alkyl.

18. Verbindung nach Anspruch 8, worin R¹ C(O)NHEt ist,
R², R³, R⁴, R⁵ und R⁶ jeweils unabhängig ausgewählt sind aus der Gruppe bestehend aus Wasserstoff-, Halogenatom, CF₃, CN, OR²⁰, C₁₋₄-Alkyl, Heterocyclyl und Aryl, worin die Alkylund Arylgruppen gegebenenfalls mit einem Arylsubstituenten substituiert sind, und
R²⁰ ausgewählt ist aus der Gruppe bestehend aus H und C₁₋₃-Alkyl.

19. Verbindung nach Anspruch 8, worin R¹ C(O)NHEt ist,
R², R³, R⁴, R⁵ und R⁶ jeweils unabhängig ausgewählt sind aus der Gruppe bestehend aus Wasserstoff-, Halogenatom, CF₃, CN, OR²⁰, Aryl, C₁₋₄-Alkyl, gegebenenfalls substituiert mit einem Substituenten, der ausgewählt ist aus Aryl und einem fusionierten 5- bis 7-gliedrigen Ring mit 1 bis 3 Heteroatomen, ausgewählt aus O, N und S, und
R²⁰ ausgewählt ist aus der Gruppe bestehend aus H und C₁₋₃-Alkyl.

20. Verbindung nach Anspruch 8, worin R¹ C(O)NHEt ist,
R², R³, R⁴, R⁵ und R⁶ jeweils unabhängig ausgewählt sind aus der Gruppe bestehend aus Wasserstoff-, Halogenatom, CF₃, CN, OR²⁰, Aryl und C₁₋₄-Alkyl, gegebenenfalls substituiert mit einem Substituenten, der ausgewählt ist aus der Gruppe bestehend aus Aryl und einem fusionierten 5-gliedrigen Ring mit zwei Sauerstoffatomen, und
R²⁰ ausgewählt ist aus der Gruppe bestehend aus H und C₁₋₃-Alkyl.

21. Verbindung nach Anspruch 8, worin R¹ C(O)NHEt ist,
R², R³, R⁴, R⁵ und R⁶ jeweils unabhängig ausgewählt sind aus der Gruppe bestehend aus Wasserstoffatom, CN, Aryl und C₁₋₄-Alkyl, gegebenenfalls substituiert mit einem Substituenten, der aus Aryl und einem fusionierten 5-gliedrigen Ring mit zwei Sauerstoffatomen ausgewählt ist, wobei sich die Verknüpfungsstellen an der 2- und 3-Position befinden.

22. Verbindung nach Anspruch 21, worin vier Substituenten aus der Gruppe bestehend aus R², R³, R⁴, R⁵ und R⁶ Wasserstoffatome sind.

23. Verbindung nach Anspruch 1, wobei die Verbindung ausgewählt ist aus der Gruppe bestehend aus (4S,2R,3R,5R)-2-{6-Amino-2-[3-(2-phenylphenoxy)prop-1-ynyl]purin-9-yl}-5-(hydroxymethyl)oxolan-3,4-diol, (4S,2R,3R,5R)-2-[6-Amino-2-(3-phenoxyprop-1-ynyl)-purin-9-yl]-5-(hydroxymethyl)oxolan-3,4-diol, 4-(3-{9-[(4S,2R,3R,5R)-3,4-Dihydroxy-5-(hydroxymethyl)oxolan-2-yl]-6-aminopurin-2-yl}prop-2-ynyloxy)benzolcarbonitril, (4S,2R,3R,5R)-2-{6-Amino-2-[3-(4-phenylphenoxy)prop-1-ynyl]purin-9-yl}-5-(hydroxymethyl)oxolan-3,4-diol, (4S,2R,3R,5R)-2-(6-Amino-2-{3-[2-benzylphenoxy]prop-1-ynyl}purin-9-yl)-5-(hydroxymethyl)oxolan-3,4-diol, 2-[2-(3-(2H-Benzo[2,3-d]-1,3-dioxolen-4-yloxy)prop-1-ynyl)-6-aminopurin-9-yl](4S,2R,3R,5R)-5-(hydroxymethyl)oxolan-3,4-diol, (4S,2R,3R,5R)-2-(6-Amino-2-{3-[3,5-bis(tert-butyl)phenoxy]prop-1-ynyl}purin-9-yl)-5-(hydroxymethyl)oxolan-3,4-diol und Gemischen davon.

24. Verwendung einer Verbindung nach Anspruch 1 für die Herstellung einer pharmazeutischen Zusammensetzung zur Stimulierung der Koronardilatation bei einem Säuger.

25. Verwendung nach Anspruch 24, wobei die pharmazeutische Zusammensetzung der Verabreichung einer therapeutisch wirksamen Menge der Verbindung nach Anspruch 1 in einem Bereich von etwa 0,01 bis etwa 100 mg/kg Gewicht des Säugers dient.

26. Verwendung nach Anspruch 24, wobei der Säuger ein Mensch ist.

27. Pharmazeutische Zusammensetzung, die die Verbindung nach Anspruch 1 und ein oder mehrere pharmazeutische Exzipienzien umfasst.

28. Pharmazeutische Zusammensetzung nach Anspruch 27, wobei die pharmazeutische Zusammensetzung in Form einer Lösung vorliegt.

29. Pharmazeutische Zusammensetzung nach Anspruch 27, wobei die Zusammensetzung als entzündungshemmendes Mittel, bei der begleitenden Therapie von Angioplastie, als Blutplättchen-Aggregationshemmstoff und als Hemmstoff der Aktivierung von Blutplättchen und Neutrophilen verwendbar ist.

30. Pharmazeutische Zusammensetzung nach Anspruch 27, die ein Pro-Pharmakon einer Verbindung nach Anspruch 1 enthält.

## Revendications

1. Composé ayant la formule: 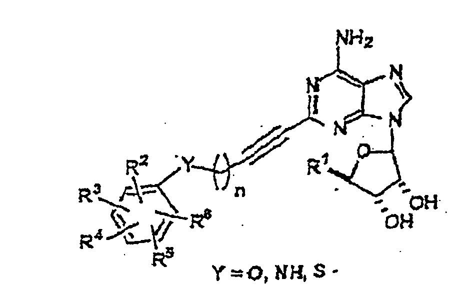 où n est 1 à 2;
Y est O, NH ou S;
R¹ est -CH₂OH ou -C(=O)NR⁷R⁸;
R², R³, R⁴, R⁵ et R⁶ sont choisis, chacun individuellement, dans le groupe consistant en hydrogène, halogéno, NO₂, CF₃, CN, OR²⁰, SR²⁰, N(R²⁰)₂, S(O)R²², SO₂R²², SO₂N(R²⁰)₂, SO₂N R²⁰COR²², SO₂NR²⁰CO₂R²², SO₂N R²⁰CON(R²⁰)₂, N(R²⁰)₂, NR²⁰COR²², NR²⁰CO₂R²², NR²⁰CON(R²⁰)₂, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰, OCON(R²⁰)₂, alkyle en C₁₋₁₅, alcényle en C₂₋₁₅, alcynyle en C₂₋₁₅, alcoxy, hétérocyclyle, aryle et hétéroaryle, tandis que les groupes alkyle, alcényle, alcynyle, alcoxy, aryle, hétérocyclyle et hétéroaryle sont en option substitués par 1 à 3 substituants choisis indépendamment dans le groupe consistant en halogéno, NO₂, hétérocyclyle, aryle, hétéroaryle, CF₃, CN, OR²⁰, SR²⁰, N(R²⁰)₂, S(O)R²², SO₂R²², SO₂N(R²⁰)₂, SO₂NR²⁰COR²², SO₂NR²⁰CO₂R²², SO₂N R²⁰CON(R²⁰)₂, N(R²⁰)₂, NR²⁰COR²², NR²⁰CO₂R²², NR²⁰CON(R²⁰)₂, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰ et OCON(R)²⁰)₂, avec chaque substituant hétéroaryle, aryle et hétérocyclyle optionnel en outre substitué en option par des groupes halogéno, NO₂, alkyle, CF₃, amino, mono- ou di-alkylamino, alkyl, aryl ou hétéroaryl amide, NCOR²², NR²⁰SO₂R²², COR²⁰, CO₂R²⁰, CON(R²⁰)₂, NR²⁰CON(R²⁰)₂, OC(O)R²⁰, OC(O)N(R²⁰)₂, SR²⁰, S(O)R²², SO₂R²², SO₂N(R²⁰)₂, CN et OR²⁰;
R⁷ et R⁸ sont, chacun individuellement, choisis parmi H et un alkyle en C₁₋₁₅ éventuellement substitué par 1 à 2 substituants, chaque substituant étant choisi individuellement dans le groupe consistant en halogéno, NO₂, hétérocyclyle, aryle, hétéroaryle, CF₃, CN, OR²⁰, SR²⁰, N(R²⁰)₂, S(O)R²², SO₂R²², SO₂N(R²⁰)₂, SO₂N R²⁰COR^{22,}, SO₂NR²⁰CO₂R^{22,}, SO₂N R²⁰CON(R²⁰)₂, N(R²⁰)₂, NR²⁰COR²², NR²⁰CO₂R²², NR²⁰CON(R²⁰)₂, COR²⁰, CO₂R²⁰, CON(R²⁰)₂, CONR²⁰SO₂R²², NR²⁰SO₂R²², SO₂NR²⁰CO₂R²², OCONR²⁰SO₂R²², OC(O)R²⁰, C(O)OCH₂OC(O)R²⁰ et OCON(R²⁰)₂, avec chaque substituant optionnel hétéroaryle, aryle et hétérocyclyle en outre substitué en option par des groupes halogéno, NO₂, alkyle, CF₃, amino, mono- ou di-alkylamino, alkyl ou aryl ou hétéroaryl amide, NCOR²², NR²⁰SO₂R²², COR²⁰, CO₂R²⁰, CON(R²⁰)₂, NR²⁰CON(R²⁰)₂, OC(O)R²⁰, OC(O)N(R²⁰)₂, SR²⁰, S(O)R²², SO₂R²², SO₂N(R²⁰)₂, CN et OR²⁰;
R²⁰ est choisi dans le groupe consistant en H, alkyle en C₁₋₁₅, alcényle en C₂₋₁₅, alcynyle en C₂₋₁₅, hétérocyclyle, aryle et hétéroaryle, tandis que les substituants alkyle, alcényle, alcynyle, hétérocyclyle, aryle et hétéroaryle sont chacun substitués en option par 1 à 3 substituants choisis indépendamment parmi halogéno, alkyle, mono-ou dialkylamino, alkyl ou aryl ou hétéroaryl amide, CN, O-alkyle enC₁₋ ₆, CF₃, aryle et hétéroaryle; et
R²² est choisi dans le groupe consistant en alkyle en C₁₋₁₅, alcényle en C₂₋₁₅, alcynyle en C₂₋₁₅, hétérocyclyle, aryle et hétéroaryle, lesquels alkyle, alcényle, alcynyle, hétérocyclyle, aryle et hétéroaryle sont chacun substitués en option par 1 à 3 substituants choisis indépendamment parmi halogéno, alkyle, mono- ou dialkylamino, alkyl ou aryl ou hétéroaryl amide, CN, O-alkyle en C₁₋₆, CF₃ et hétéroaryle.

2. Composé selon la revendication 1, dans lequel R², R³, R⁴, R⁵ et R⁶ sont, chacun individuellement, choisis dans le groupe consistant en hydrogène, halogéno, NO₂, CF₃, CN, OR²⁰, SR²⁰, N(R²⁰)₂, S(O)R²², SO₂R²², SO₂N(R²⁰)₂, NR²⁰CO₂R²², NR²⁰CON(R²⁰)₂, COR²⁰, CO₂R²⁰, CON(R²⁰)₂, NR²⁰SO₂R²², OC(O)R²⁰, OCON(R²⁰)₂, alkyle en C₁₋₁₅, alcényle en C₂₋₁₅, alcynyle en C₂₋₁₅, hétérocyclyle, aryle et hétéroaryle, tandis que les groupes alkyle, alcényle, alcynyle, aryle, hétérocyclyle et hétéroaryle sont chacun en option substitués par 1 à 3 substituants choisis indépendamment dans le groupe consistant en halogéno, NO₂, hétérocyclyle, aryle, hétéroaryle, CF₃, CN, OR²⁰, SR²⁰, N(R²⁰)₂, S(O)R²², SO₂R²², SO₂N(R²⁰)₂, NR²⁰C O₂R²², NR²⁰CON(R²⁰)₂, COR²⁰, CO₂R²⁰, CON(R²⁰)₂, NR²⁰S O₂R²², OC(O)R²⁰ et OCON(R)²⁰)₂, avec chaque substituant hétéroaryle, aryle et hétérocyclyle optionnel en outre substitué en option par des groupes halogéno, alkyle, CF₃, mono- ou di-alkylamino, SR²⁰, CN et OR²⁰;
R⁷ et R⁸ sont, chacun individuellement, choisis parmi H et un alkyle en C₁₋₁₅ éventuellement substitué par 1 à 2 substituants choisis indépendammment dans le groupe consistant en aryle, hétéroaryle, CF₃ et OR²⁰, tandis que chaque substituant optionnel hétéroaryle ou aryle est substitué en option par des groupes halogéno, alkyle et CF₃; et
R²⁰ est choisi dans le groupe consistant en H, alkyle en C₁₋₆, alcényle en C₂₋₆, alcynyle en C₂₋₆ et aryle, tandis que chacun des alkyle, alcényle, alcynyle et aryle sont en outre substitués en option par 1 à 3 substituants choisis indépendamment parmi halogéno, alkyle, CN, O-alkyle enC₁₋₆ et CF₃.

3. Composé selon la revendication 1, dans lequel R², R³, R⁴, R⁵ et R⁶ sont choisis individuellement dans le groupe consistant en hydrogène, halogéno, NO₂, CF₃, CN, OR²⁰, SR²⁰, N(R²⁰)₂, alkyle en C₁₋₁₅, alcényle en C₂₋₁₅, hétérocyclyle, aryle et hétéroaryle, lesquels groupes alkyle, alcényle, aryle, hétérocyclyle et hétéroaryle sont en option substitués par 1 à 3 substituants choisis indépendamment dans le groupe consistant en halogéno, NO₂, aryle, CF₃, CN, OR²⁰, SR²⁰, N(R²⁰)₂, avec chaque substituant aryle optionnel en outre substitué en option par des groupes halogéno, alkyle, CF₃, SR²⁰, CN et OR²⁰;
R⁷ et R⁸ sont, chacun indépendamment, choisis parmi H et un alkyle en C₁₋₁₅ éventuellement substitué par 1 à 2 groupes CF₃; et
R²⁰ est choisi dans le groupe consistant en H et un alkyle en C₁₋₄, tandis que l'alkyle en C₁-C₄ est substitué en option par 1 à 3 substituants choisis indépendamment prmi halogéno, alkyle, CN, O-alkyle enC₁₋₃ et CF₃.

4. Composé selon la revendication 1, dans lequel R², R³, R⁴, R⁵ et R⁶ sont chacun choisis individuellement dans le groupe consistant en hydrogène, halogéno, CF₃, CN, OR²⁰, SR²⁰, N(R²⁰)₂, alkyle en C₁₋₁₅, hétérocyclyle et aryle, tandis que les groupes alkyle, aryle et hétérocyclyle sont en option substitués par 1 à 2 substituants choisis indépendamment dans le groupe consistant en halogéno, aryle, CF₃, CN, OR²⁰, SR²⁰, N(R²⁰)₂, et tandis que chaque substituant aryle optionnel est en outre substitué en option par des groupes halogéno, alkyle, CF₃, CN et OR²⁰;
R⁷ et R⁸ sont, chacun individuellement, choisis parmi H et un alkyle en C₁₋₁₅; et
R²⁰ est choisi dans le groupe consistant en H et un alkyle en C₁₋₃, tandis que l'alkyle en C₁-C₃ est substitué en option par 1 à 3 substituants choisis indépendamment prmi halogéno, alkyle, CN et CF₃.

5. Composé selon la revendication 1, dans lequel R², R³, R⁴, R⁵ et R⁶ sont chacun choisis individuellement dans le groupe consistant en hydrogène, halogéno, CF₃, CN, OR²⁰, SR²⁰ et N(R²⁰)₂, alkyle en C₁₋ ₁₅, hétérocyclyle et aryle, tandis que les groupes alkyle, aryle et hétérocyclyle sont en option substitués par 1 à 2 substituants choisis indépendamment dans le groupe consistant en halogéno, aryle, CF₃, CN, OR²⁰, SR²⁰ et N(R²⁰)₂, et tandis que chaque substituant aryle optionnel est en outre substitué en option par des groupes halogéno, alkyle, CN et CF₃;
R⁷ et R⁸ sont, chacun individuellement, choisis parmi H et un alkyle en C₁₋₈; et
R²⁰ est choisi dans le groupe consistant en H et un alkyle en C₁₋₃.

6. Composé selon la revendication 5, dans lequel au moins un substituant choisi dans le groupe consistant en R², R³, R⁴, R⁵ et R⁶ est l'hydrogène.

7. Composé selon la revendication 5, dans lequel au moins deux substituants choisis dans le groupe consistant en R², R³, R⁴, R⁵ et R⁶ sont l'hydrogène.

8. Composé selon la revendication 5, dans lequel au moins trois substituants choisis dans le groupe consistant en R², R³, R⁴, R⁵ et R⁶ sont l'hydrogène, tandis que Y est O et tandis que n est 1.

9. Composé selon la revendication 8, dans lequel R¹ est CH₂OH;
R², R³, R⁴, R⁵ et R⁶ sont chacun individuellement choisis dans le groupe consistant en hydrogène, halogéno, CF₃, CN, OR²⁰, alkyle en C₁₋₈, hétérocyclyle et aryle, lesquels groupes alkyle, aryle et hétérocyclyle sont en option substitués par un substituant choisi dans le groupe consistant en halogéno, aryle, CF₃, CN et OR²⁰, et tandis que chaque substituant aryle optionnel est en outre substitué en option par des groupes halogéno, alkyle, CN et CF₃; et
R²⁰ est choisi dans le groupe consistant en H et un alkyle en C₁₋₃.

10. Composé selon la revendication 8, dans lequel R¹ est CH₂OH;
R², R³, R⁴, R⁵ et R⁶ sont chacun individuellement choisis dans le groupe consistant en hydrogène, halogéno, CF₃, CN, OR²⁰, alkyle en C₁₋₆, hétérocyclyle et aryle, ltandis que les groupes alkyle et aryle sont en option substitués par un substituant choisi dans le groupe consistant en halogéno, aryle, CF₃, CN et OR²⁰; et
R²⁰ est choisi dans le groupe consistant en H et un alkyle en C₁₋₃.

11. Composé selon la revendication 8, dans lequel R¹ est CH₂OH;
R², R³, R⁴, R⁵ et R⁶ sont chacun individuellement choisis dans le groupe consistant en hydrogène, halogéno, CF₃, CN, OR²⁰, alkyle en C₁₋₄, hétérocyclyle et aryle, tandis que les groupes alkyle et aryle sont en option substitués par un aryle; et
R²⁰ est choisi dans le groupe consistant en H et un alkyle en C₁₋₃.

12. Composé selon la revendication 8, dans lequel R¹ est CH₂OH;
R², R³, R⁴, R⁵ et R⁶ sont chacun individuellement choisis dans le groupe consistant en hydrogène, halogéno, CF₃, CN, OR²⁰, alkyle en C₁₋₄ substitué en option par un groupe aryle et hétérocyclyle, tandis que le groupe hétérocyclyle est un cycle condensé à cinq à sept chaînons contenant 1 à 3 hétéroatomes choisis dans le groupe consistant en O, N, S et leurs combinaisons; et
R²⁰ est choisi dans le groupe consistant en H et un alkyle en C₁₋₃.

13. Composé selon la revendication 8, dans lequel R¹ est CH₂OH;
R², R³, R⁴, R⁵ et R⁶ sont chacun individuellement choisis dans le groupe consistant en hydrogène, halogéno, CF₃, CN, OR²⁰, alkyle en C₁₋₄ et aryle substitué en option par un groupe aryle et hétérocyclyle, tandis que le groupe hétérocyclyle est un cycle condensé à cinq chaînons contenant 2 atomes d'oxygène; et
R²⁰ est choisi dans le groupe consistant en H et un alkyle en C₁₋₃.

14. Composé selon la revendication 8, dans lequel R¹ est CH₂OH, et R², R³, R⁴, R⁵ et R⁶ sont chacun individuellement choisis dans le groupe consistant en hydrogène, CN, et alkyle en C₁₋₄ substitué en option par un groupe aryle et un cycle condensé à cinq chaînons contenant deux atomes d'oxygène avec des points de fixation sur la position 2 et 3.

15. Composé selon la revendication 14, dans lequel quatre substituants choisis dans le groupe consistant en R², R³, R⁴, R⁵ et R⁶ sont l'hydrogène.

16. Composé selon la revendication 8, dans lequel R¹ est C(O)NHEt;
R², R³, R⁴, R⁵ et R⁶ sont chacun individuellement choisis dans le groupe consistant en hydrogène, halogéno, CF₃, CN, OR²⁰, alkyle en C₁₋₈, hétérocyclyle et aryle, tandis que les groupes alkyle, aryle et hétérocyclyle sont en option substitués par un substituant choisi indépendamment dans le groupe consistant en halogéno, aryle, CF₃, CN et OR²⁰, et tandis que chaque substituant aryle optionnel est en outre substitué en option par des groupes halogéno, alkyle, CN et CF₃; et
R²⁰ est choisi dans le groupe consistant en H et un alkyle en C₁₋₃.

17. Composé selon la revendication 8, dans lequel R¹ est C(O)NHEt;
R², R³, R⁴, R⁵ et R⁶ sont chacun individuellement choisis dans le groupe consistant en hydrogène, halogéno, CF₃, CN, OR²⁰, alkyle en C₁₋₆, hétérocyclyle et aryle, tandis que les groupes alkyle et aryle sont en option substitués par un substituant choisi dans le groupe consistant en halogéno, aryle, CF₃, CN et OR²⁰; et
R²⁰ est choisi dans le groupe consistant en H et un alkyle en C₁₋₃.

18. Composé selon la revendication 8, dans lequel R¹ est C(O)NHEt;
R², R³, R⁴, R⁵ et R⁶ sont chacun individuellement choisis dans le groupe consistant en hydrogène, halogéno, CF₃, CN, OR²⁰, alkyle en C₁₋₄, hétérocyclyle et aryle, tandis que les groupes alkyle et aryle sont en option substitués par un substituant aryle; et
R²⁰ est choisi dans le groupe consistant en H et un alkyle en C₁₋₃.

19. Composé selon la revendication 8, dans lequel R¹ est C(O)NHEt;
R², R³, R⁴, R⁵ et R⁶ sont chacun individuellement choisis dans le groupe consistant en hydrogène, halogéno, CF₃, CN, OR²⁰, aryle, alkyle en C₁₋₄ substitué en option par un substituant choisi parmi un aryle, et un cycle condensé à cinq à sept chaînons contenant 1-3 hétéroatomes choisis dans le groupe consistant en O, N, S; et
R²⁰ est choisi dans le groupe consistant en H et un alkyle en C₁₋₃.

20. Composé selon la revendication 8, dans lequel R¹ est C(O)NHEt;
R², R³, R⁴, R⁵ et R⁶ sont chacun individuellement choisis dans le groupe consistant en hydrogène, halogéno, CF₃, CN, OR²⁰, aryle, et alkyle en C₁₋₄ qui est substitué en option par un substituant choisi dans le groupe consistant en aryle et un cycle condensé à cinq chaînons contenant deux atomes d'oxygène; et
R²⁰ est choisi dans le groupe consistant en H et un alkyle en C₁₋₃.

21. Composé selon la revendication 8, dans lequel R¹ est C(O)NHEt;
R², R³, R⁴, R⁵ et R⁶ sont chacun individuellement choisis dans le groupe consistant en hydrogène, CN, aryle, et alkyle en C₁₋₄ qui est substitué en option par un substituant choisi parmi un aryle et un cycle condensé à cinq chaînons contenant deux atomes d'oxygène avec des points de fixation sur la position 2 et 3.

22. Composé selon la revendication 21, dans lequel quatre substituants choisis dans le groupe consistant en R², R³, R⁴, R⁵ et R⁶ sont l'hydrogène.

23. Composé selon la revendication 1, dans lequel le composé est choisi dans le groupe consistant en (4S,2R,3R,5R)-2-{6-amino-2-[3-(2-phénylphénoxy)prop-1-ynyl]purine-9-yl}-5-(hydroxyméthyl)oxolane-3,4-diol, (4S,2R,3R,5R)-2-[6-amino-2-(3-phénoxyprop-1-ynyl)purine-9-yl]-5-(hydroxyméthyl)oxolane-3,4-diol, 4-(3-{9-[(4S,2R,3R,5R)-3,4-dihydroxy-5-(hydroxyméthyl)oxolane-2-yl]-6-aminopurine-2-yl}prop-2-ynyloxy)benzènecarbonitrile, (4S,2R,3R,5R)-2-{6-amino-2-[3-(4-phénylphénoxy)prop-1-ynyl]purine-9-yl}-5-(hydroxyméthyl)oxolane-3,4-diol, (4S,2R,3R,5R)-2-(6-amino-2-{3-[2-benzylphénoxy]prop-1-ynyl}purine-9-yl)-5-(hydroxyméthyl)oxolane-3,4-diol, 2-[2-(3-(2H-benzo[2,3-d]1,3-dioxolène-4-yloxy)prop-1-ynyl)-6-aminopurine-9-yl](4S,2R,3R,5R)-5-(hydroxyméthyl)oxolane-3,4-diol, (4S,2R,3R,5R)-2-(6-amino-2-{3-[3,5-bis(tert-butyl)phénoxy]prop-1-ynyl}purine-9-yl)-5-(hydroxyméthyl)oxolane-3,4-diol, et leurs mélanges.

24. Utilisation d'un composé selon la revendication 1 pour la préparation d'une composition pharmaceutique pour la stimulation de la vasodilatation coronarienne chez un mammifère.

25. Utilisation selon la revendication 24, dans laquelle la composition pharmaceutique est pour administration d'une quantité thérapeutiquement efficace d'un composé selon la revendication I allant d'environ 0,01 à environ 100 mg/kg de poids du mammifère.

26. Utilisation selon la revendication 24, dans laquelle le mammifère est un humain.

27. Composition pharmaceutique comprenant le composé selon la revendication 1 et un ou plusieurs excipients pharmaceutiques.

28. Composition pharmaceutique selon la revendication 27, dans laquelle la composition pharmaceutique est sous la forme d'une solution.

29. Composition pharmaceutique selon la revendication 27, dans laquelle la composition pharmaceutique est utile comme anti-inflammatoire, en traitement d'appoint avec l'angioplastie, comme inhibiteur d'agrégation plaquettaire, et comme inhibiteur d'activation plaquettaire et neutrophile.

30. Composition pharmaceutique selon la revendication 27, comportant un promédicament d'un composé selon la revendication 1.
